(19) Europäisches Patentamt / European Patent Office / Office européen des brevets

(11) **EP 2 435 042 B1**

(12) **FASCICULE DE BREVET EUROPEEN**

(45) Date de publication et mention
de la délivrance du brevet:
**20.12.2017 Bulletin 2017/51**

(21) Numéro de dépôt: **10728845.8**

(22) Date de dépôt: **28.05.2010**

(51) Int Cl.:
*A61K 9/50* (2006.01)  *A61K 9/00* (2006.01)
*A61K 31/403* (2006.01)  *B01J 13/04* (2006.01)

(86) Numéro de dépôt international:
**PCT/FR2010/051039**

(87) Numéro de publication internationale:
**WO 2010/136740 (02.12.2010 Gazette 2010/48)**

(54) **PROCEDE DE PREPARATION DE PARTICULES CREUSES ET LEURS APPLICATIONS**

VERFAHREN ZUR HERSTELLUNG VON HOHLEN PARTIKELN UND IHRE VERWENDUNGEN

METHOD FOR PREPARING HOLLOW PARTICLES, AND USES THEREOF

(84) Etats contractants désignés:
**AL AT BE BG CH CY CZ DE DK EE ES FI FR GB
GR HR HU IE IS IT LI LT LU LV MC MK MT NL NO
PL PT RO SE SI SK SM TR**

(30) Priorité: **29.05.2009 FR 0953607
29.05.2009 US 182533 P**

(43) Date de publication de la demande:
**04.04.2012 Bulletin 2012/14**

(73) Titulaire: **Flamel Ireland Limited
Dublin 15 (IE)**

(72) Inventeurs:
• **CASTAN, Catherine
69510 Souci En Jarrest (FR)**
• **CAISSE, Philippe
69720 St Bonnet De Mure (FR)**

(74) Mandataire: **Cabinet Plasseraud
235 Cours Lafayette
69006 Lyon (FR)**

(56) Documents cités:
**WO-A-97/22409**      **WO-A-2009/087665**
**FR-A- 2 842 736**    **US-A- 3 976 764**
**US-A- 5 057 323**    **US-A- 5 626 876**
**US-A1- 2002 172 716**

• **DATABASE WPI Week 200810 Thomson
Scientific, London, GB; AN 2007-026684
XP002561177 & CN 1 331 590 C (UNIV ZHEJIANG)
15 août 2007 (2007-08-15)**

EP 2 435 042 B1

**Description**

**DOMAINE DE L'INVENTION**

La présente demande concerne un procédé de préparation de particules creuses. Elle concerne également lesdites particules et leurs applications, en particulier dans la fabrication de systèmes flottants à libération contrôlée de principe actif, notamment de principe actif pharmaceutique.

**ETAT DE LA TECHNIQUE**

Il a déjà été envisagé de préparer des particules de faibles densités pour diverses applications, et notamment pour des applications en pharmacie pour des systèmes pharmaceutiques gastro-rétentifs.

Deux approches sont envisagées, la première étant la réalisation de particules poreuses et la seconde la réalisation de particules creuses.

Ainsi, par extrusion sphéronisation (WO 91/18590), on a pu préparer des matrices poreuses de cellulose. En utilisant une poudre de polypropylène mousse, A. Streubel et al. (International Journal of Pharmaceutics 241 (2002) 279-292, ont préparé des microparticules par évaporation de solvant. La même technique a été utilisée avec du polycarbonate [N.J. Joseph et al., Journal of Controlled Release 79 (2002) 71-79]. En utilisant une suspension de cristaux de NaCl ou sucrose dans du polyacide lactique dans une tour d'atomisation à disque tournant (spinning disk atomisation) des gouttelettes sphériques sont formées par centrifugation et le solvant (chloroforme) dissout les cristaux, formant ainsi des microparticules poreuses [Y. Senuma, Biomaterials 21 (2000) 1135-1144]. Ces méthodes mettent en oeuvre des solvants chlorés.

Des microparticules de poly(D,L-lactide-co-glycolide) poreuses ont été préparées par atomisation électrodynamique (electrodynamic atomisation) [Jingwey Xie et al., Journal of Colloid and Interface Science, 302 (206) 103-112].

Le document WO 97/22409 décrit un procédé de préparation de microcapsules de polymère comportant de multiples pores pouvant être remplis de gaz. Ce procédé comprend les étapes suivantes : (i) dissolution d'un polymère filmogène dans un solvant volatile non aqueux pour former une solution de polymère, (ii) dispersion de particules finement divisées d'un matériau solide dans la solution de polymère obtenue à l'étape (i) pour former une suspension de particules solides, (iii) évaporation du solvant pour former un enrobage polymérique solide sur les particules de matériau solide afin de produire des microcaspules de polymères encapsulant les particules de matériau solide, (iv) récupération des microcaspules de polymères et (v) extraction du matériau solide encapsulé des microcaspules de polymères.

Par ces techniques, il est difficile d'obtenir des particules présentant une forme homogène et présentant une porosité reproductible qui soit essentiellement une porosité fermée. Afin de pallier à ces difficultés, il a été envisagé de préparer plutôt que des particules poreuses, des particules creuses.

Ainsi, des procédés de préparation de billes d'alginate de calcium par des techniques de gélification ionotropique (R.Talukder et R. Fassihi, Drug Development and Industrial Pharmacy, vol.30, n°4 (2004) p.405-412), ou de type grelonnage (prilling) (Y. Murata et al., European Journal of Pharmaceutics and Biopharmaceutics, 50 (2000) p.221-226) ont été décrits. Les billes obtenues ne présentent pas toutes une bonne sphéricité et résistance mécanique. Elles présentent non pas une seule cavité centrale mais plutôt plusieurs cavités dont la taille est fonction des paramètres du procédé. Plus la taille des cavités est importante, plus leur forme est aléatoire et moins leur résistance mécanique est importante. WO 99/20254 décrit la préparation de microsphères creuses, en l'absence de tout solvant chloré, par émulsion-diffusion de solvant. Cependant, il est difficile de régler tous les paramètres du procédé pour obtenir exclusivement des particules creuses, sans produire conjointement des particules poreuses, voire des particules pleines.

Il existe donc un réel besoin en un procédé permettant la préparation de particules creuses qui présentent des caractéristiques de forme homogènes, une répartition contrôlée de taille et de bonnes caractéristiques de résistance mécanique. En outre, de telles particules peuvent présenter de bonnes caractéristiques de flottabilité.

**BREVE DESCRIPTION DE L'INVENTION**

Il est du mérite des inventeurs d'avoir trouvé un procédé simple de préparation de particules creuses présentant l'ensemble des caractéristiques souhaitées.

L'invention concerne également lesdites particules creuses ainsi que leurs applications, notamment dans la fabrication de systèmes flottants permettant la libération de substances actives déposées à leur surface. L'invention concerne également lesdits systèmes flottants et des compositions pharmaceutiques à base de tels systèmes flottants.

**BREVE DESCRIPTION DES FIGURES**

- La figure 1 représente de façon schématique les différentes étapes A à F de préparation des microparticules de matière active à libération contrôlée de l'invention.
- Les Figures 2a et 2b représentent des photographies au microscope électronique à balayage (grossissement x 100) d'une microparticule pleine de l'exemple 1, respectivement en vue de dessus et en coupe transversale.
- Les Figures 3a et 3b représentent des photographies au microscope électronique à balayage (grossissement x 100) d'une particule creuse de l'exemple 1, respectivement en vue de dessus et en coupe transversale.
- La Figure 4 représente une photographie au microscope binoculaire (grossissement 1,6 x 1,6) de particules pleines obtenues à l'exemple 2.
- La Figure 5 représente une photographie au microscope binoculaire (grossissement 1,6 x 1,6) de supports creux obtenus à l'exemple 2.
- La Figure 6 représente une photographie au microscope binoculaire (grossissement 1,6 x 1,6) de particules pleines de l'exemple 3.
- La Figure 7 représente une photographie au microscope binoculaire (grossissement 1,6 x 1,6) de supports creux obtenus à l'exemple 3.
- La Figure 8 représente une photographie au microscope binoculaire (grossissement 1,6 x 1,6) de particules à libération prolongée obtenues à l'exemple 4.
- La Figure 9 représente un graphique représentant la fraction de particules selon l'exemple 3 (triangle noir) et de supports creux préparés selon l'exemple 2 (carré blanc) et l'exemple 3 (triangle blanc) qui restent à la surface du milieu liquide en fonction du temps.
- La Figure 10 représente un graphique représentant la fraction de particules ou de supports creux qui restent à la surface du milieu liquide en fonction du temps selon l'exemple 4 (carré noir), l'exemple 5 (carré blanc), l'exemple 6 (losange noir) et l'exemple 7 (triangle noir).
- La Figure 11 représente un graphique représentant le profil de dissolution in vitro de particules à libération contrôlée de carvédilol phosphate de l'exemple 4.
- La Figure 12 représente un graphique représentant le profil de dissolution in vitro de particules à libération contrôlée de carvédilol phosphate de l'exemple 5.
- La Figure 13 représente un graphique représentant le profil de dissolution in vitro de particules à libération contrôlée de l'exemple 6.
- La Figure 14 représente un graphique représentant le profil de dissolution in vitro de particules à libération contrôlée de l'exemple 7.
- La Figure 15 représente une photographie au microscope binoculaire (grossissement 3.2 x 1,6) des supports creux obtenus à l'exemple 8.
- La Figure 16 représente un graphique représentant le profil de dissolution in vitro de particules à libération contrôlée de l'exemple 8.
- La Figure 17 représente une photographie au microscope binoculaire (grossissement 3.2 x 1,6) des particules pleines obtenues à l'exemple 9.
- La Figure 18 représente une photographie au microscope binoculaire (grossissement 3.2 x 1,6) des supports creux obtenus à l'exemple 9.
- La Figure 19 représente les substrats d'acide acétyl salicylique utilisés à l'exemple 10.
- La Figure 20 représente une photographie au microscope binoculaire (grossissement 1,6 x 1,6) des particules pleines obtenues à l'exemple 10.
- La Figure 21 représente une photographie au microscope binoculaire (grossissement 1,6 x 1,6) de supports creux obtenus à l'exemple 10.
- La Figure 22 représente les substrats d'acide acétyl salicylique utilisés à l'exemple 11.
- La Figure 23 représente une photographie au microscope binoculaire (grossissement 1,6 x 1,6) des particules pleines obtenues à l'exemple 11.
- La Figure 24 représente une photographie au microscope binoculaire (grossissement 1,6 x 1,6) de supports creux obtenus à l'exemple 11.
- La Figure 25 représente une photographie au microscope binoculaire (grossissement 1,6 x 1,6) des particules pleines obtenues à l'exemple 16.
- La Figure 26 représente une photographie au microscope binoculaire (grossissement 1,6 x 1,6) de supports creux obtenus à l'exemple 17.

## DESCRIPTION DETAILLEE DE L'INVENTION

### Définitions

Par "masse volumique" il est entendu la masse volumique apparente après tassement, selon la définition de la pharmacopée européenne (chapitre 02.09.34 édition 6.4). La masse volumique apparente d'une poudre est la masse moyenne par unité de volume, ce volume incluant la fraction solide de la poudre, la porosité intra granulaire et la porosité inter granulaire. Cette valeur dépendant de la méthode de mesure, la méthode utilisée est décrite ci-dessous en relation avec le chapitre TESTS.

Au sens de l'invention et dans tout le présent exposé, le terme « principe actif » couvre non seulement le principe actif en tant que tel mais aussi ses sels, ses énantiomères, ses isomères, ses solvates ou ses formes polymorphes.

On utilise ou bien le terme « enrobage » ou bien le terme « pelliculage » pour désigner une couche recouvrant de façon complète et homogène le support ou la particule.

L'enrobage est dit « à libération contrôlée » quand il permet de modifier, retarder ou prolonger la libération du principe actif. De tels enrobages ont largement été décrits dans la littérature et ceux qui sont tout particulièrement adaptés à l'invention sont ceux décrits notamment dans les demandes de brevet EP 709 087, WO 03/030878, EP 1 524 968, EP 1 524 969 et PCT/FR2009/050719.

On entend par « particule creuse », une particule comprenant un enrobage qui délimite une partie centrale interne comprenant une unique cavité vide dont le volume correspond à au moins 20% du volume de la zone centrale, de préférence au moins 30%, de préférence au moins 40%, de préférence au moins 50%, de préférence au moins 60%, de préférence au moins 70%, de préférence au moins 80%, de préférence au moins 90%, de préférence environ 100% du volume de la partie centrale.

On entend par « cavité vide » une cavité qui contient essentiellement de l'air.

**Procédé de préparation des particules creuses :**

L'invention porte sur un procédé de préparation pour obtenir des particules creuses, lesdites particules creuses possédant une unique cavité vide entourée d'un enrobage, comprenant :

1/ le dépôt par pulvérisation liquide, notamment en lit d'air fluidisé, sur un substrat partiellement soluble dans un milieu liquide M d'un enrobage composé de :

- 40 à 100% en poids, de préférence de 70 à 100% en poids d'au moins un composé insoluble dans l'eau à pH inférieur à 5, ledit composé insoluble dans l'eau à pH inférieur à 5 comprenant au moins un polymère, et
- 60 à 0%, de préférence de 30 à 0% en poids d'au moins un composé soluble dans l'eau afin d'obtenir une particule comprenant ledit substrat enrobé d'une couche d'enrobage,

2/ l'extraction dans le milieu M d'une partie du substrat, et
3/ le séchage des particules obtenues à l'étape 2.

Par « partiellement soluble dans le milieu M » on entend un substrat dont au moins 50% en poids, de préférence au moins 75% et plus préférentiellement au moins 85% en poids peut être solubilisé dans le milieu M. De façon avantageuse, 50% en poids du substrat est dissous en moins de 48 h, de préférence en moins de 24 h et plus préférentiellement encore en moins de 12 h. Bien entendu, ce degré de solubilité pourra être modifié en faisant varier la température du milieu M dans lequel le substrat est partiellement dissous.

Le dépôt d'enrobage réalisé à l'étape 1) est un dépôt physique, c'est-à-dire que l'enrobage est appliqué sans faire intervenir de nouvelles liaisons covalentes. Ainsi le dépôt physique de l'enrobage ne comprend pas le dépôt par polymérisation.

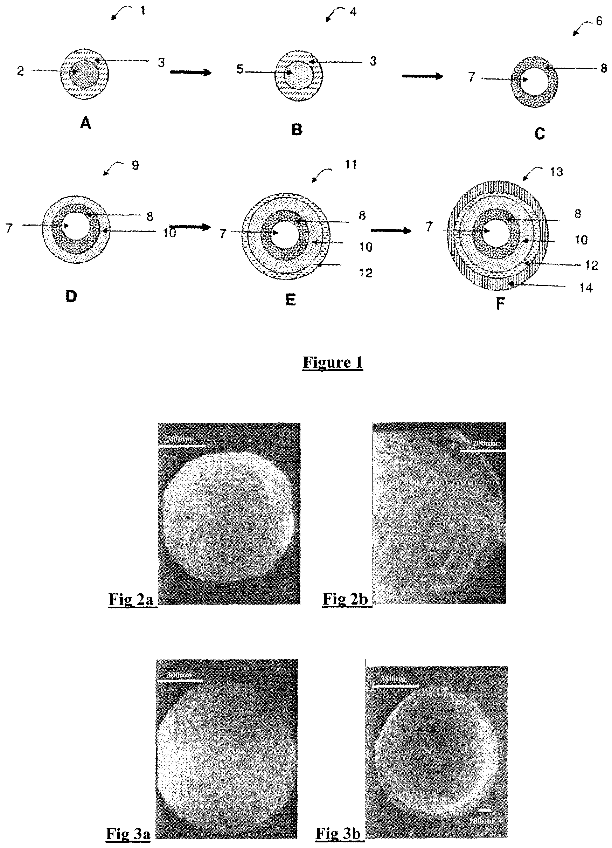

Le procédé de préparation des particules creuses conforme à l'invention est représenté schématiquement sur la figure 1, sur laquelle, les produits représentés par les lettres A, B et C représentent respectivement des produits obtenus à l'issue des étapes 1/, 2/ et 3/.

Ainsi, la particule pleine 1, obtenue à l'issue de l'étape 1/, comprend un substrat 2 enrobé par une couche 3. Le substrat est ensuite extrait et le produit obtenu 4 est représenté en B. Ce produit 4 comprend en sa partie centrale 5 délimitée par la couche 3, le milieu d'extraction M. Ce produit 4 est ensuite séché dans l'étape 3 du procédé selon l'invention pour donner en C, une particule creuse 6 comprenant une enveloppe ou enrobage 8, délimitant une cavité centrale 7 vide. Le substrat est un substrat solide, qui peut se présenter sous n'importe quelle forme géométrique. De préférence, le substrat est sensiblement sphérique.

Il est ainsi décrit un procédé de préparation d'une particule creuse 6, qui comprend les étapes suivantes :

1) dépôt physique sur un substrat 2 partiellement soluble dans un milieu liquide M d'un enrobage 3 composé de :

- 40 à 100% en poids, de préférence de 70 à 100% en poids d'au moins un composé insoluble dans l'eau à pH inférieur à 5, ledit composé insoluble dans l'eau à pH inférieur à 5 comprenant au moins un polymère, et
- 60 à 0%, de préférence de 30 à 0% en poids d'au moins un composé soluble dans l'eau,

pour obtenir une particule pleine 1 comprenant un substrat 2 enrobé d'une couche 3,

2/ Mise en contact de ladite particule 1 avec ledit liquide M de manière à extraire par dissolution tout ou partie du substrat hors de la particule 1 afin d'obtenir une particule 4 comprenant une partie centrale 5 dans laquelle le substrat extrait est remplacé par du liquide d'extraction M et qui est délimitée par la couche 3,

3/ Séchage de la particule 4 pour éliminer le liquide d'extraction M de la partie centrale 5 et ainsi obtenir une particule creuse 6 possédant une unique cavité vide 7 entourée d'un enrobage 8.

Selon l'invention, le dépôt physique de l'enrobage 3 sur le substrat 2 est réalisé par pulvérisation liquide (spray coating) de préférence en lit d'air fluidisé.

De préférence, la mise en contact se fait par immersion de la particule 1 dans le liquide M. Dans la pratique, le dépôt physique de l'enrobage se fait par pulvérisation liquide de la solution ou de la suspension d'enrobage sur une pluralité ou un ensemble de particules individuelles de substrat.

Selon un mode de réalisation particulier, le procédé selon l'invention est mis en oeuvre en utilisant des substrats choisis parmi des substances de grade pharmaceutique, qui comprennent notamment les matériaux choisis dans le groupe comprenant le saccharose, le sucrose, les sphères de sucre, le mannitol, le maltitol, le lactose, le chlorure de sodium, le chlorure de potassium, et leurs mélanges. D'autres éléments peuvent entrer dans la composition des substrats, notamment des charges, agents de compression, lubrifiants, colorants, liants, désintégrants, etc.

De préférence, le diamètre moyen équivalent en volume mesuré à 0,1 bar du substrat utilisé dans le procédé selon l'invention est supérieur ou égal à 50 $\mu$m, de préférence compris entre 50 $\mu$m et 1000 $\mu$m, ou encore préférentiellement compris entre 50 $\mu$m et 500 $\mu$m.

Le milieu M peut être n'importe quel milieu liquide. On préférera utiliser un milieu de grade alimentaire, cosmétique ou pharmaceutique de façon à pouvoir utiliser les particules creuses obtenues dans de tels domaines techniques.

Selon un mode de réalisation particulier de l'invention, le milieu M est un milieu aqueux, de préférence dépourvu de solvant organique. L'utilisation d'un milieu M aqueux comme milieu d'extraction est tout à fait avantageuse pour des raisons évidentes de coût et de disponibilité.

Le milieu M permet la dissolution au moins partielle du substrat. De préférence, lors de l'étape 2) du procédé de l'invention, au moins 20% en poids, de préférence au moins 30%, de préférence au moins 40%, de préférence au moins 50%, de préférence au moins 60%, de préférence au moins 70%, de préférence au moins 80%, de préférence au moins 90%, de préférence environ 100% en poids du substrat est extrait hors de la particule 1.

Le milieu M peut avantageusement être additionné d'un tensio-actif, notamment d'un tensio-actif anionique, non ionique ou cationique. Un tel tensio-actif est mis en oeuvre pour augmenter la mouillabilité des particules enrobées de façon à favoriser leur perméabilité et donc l'extraction par dissolution du substrat. Il permet en outre de limiter l'agrégation des particules enrobées entre elles.

En fonction du type d'enrobage et du substrat choisis, l'homme du métier est en mesure de choisir le milieu M et le tensio-actif les plus appropriés. Bien entendu, on préférera utiliser des grades de tensio-actifs adaptés à l'utilisation finale envisagée.

Afin d'optimiser la vitesse d'extraction du substrat, le milieu M peut être chauffé si besoin, ou bien on peut en modifier le pH si la solubilité du substrat est pH dépendante.

La dissolution du substrat pourra être totale, mais il est également possible de stopper la dissolution avant la dissolution complète de façon à limiter la durée de cette étape du procédé de préparation.

La durée de l'extraction du substrat à l'étape 2) du procédé de l'invention devra être suffisante pour que l'on obtienne des particules creuses, c'est-à-dire des particules présentant une cavité centrale unique telle que définie précédemment.

Selon un mode de réalisation particulier de l'invention, l'une des caractéristiques des particules creuses obtenues selon le procédé de l'invention est leur capacité à flotter dans le milieu dans lequel elles sont mises en oeuvre. Ainsi, la dissolution du substrat devra être suffisante pour obtenir des particules creuses ayant la masse volumique appropriée pour permettre leur flottaison.

La quantité d'enrobage appliquée sur le substrat doit être suffisante pour permettre aux particules creuses obtenues de présenter une résistance mécanique suffisante. De façon avantageuse, la quantité d'enrobage devra en outre être assez faible pour que lesdites particules creuses puissent flotter dans le milieu dans lequel elles seront utilisées.

La quantité d'enrobage est définie par le taux d'enrobage ou taux de pelliculage $TP_1$ des substrats qui est calculé de la façon suivante

$$TP_1 = \frac{\text{poids de l'enrobage}}{\text{poids des particules enrobées}} \times 100$$

les valeurs étant exprimées par rapport à la matière sèche.

Ce taux d'enrobage $TP_1$ est compris entre 5 et 90%, de préférence 10 à 60% et de préférence 15 à 40%. Si l'on se réfère à la figure 1, ce taux d'enrobage est égal au poids de la couche 3 divisé par le poids de la particule 1, l'ensemble étant calculé par rapport à la matière sèche des différents composants.

Ce taux d'enrobage $TP_1$ est tel que lesdites particules creuses présentent une masse volumique inférieure ou égale à 0,6 g/mL, de préférence inférieure ou égale à 0,5 g/mL, et plus préférentiellement encore inférieure ou égale à 0,4 g/mL.

De préférence, l'épaisseur de l'enrobage de la particule creuse selon l'invention est supérieure ou égale à 5 $\mu$m, de préférence supérieure ou égale à 10 $\mu$m.

D'autres propriétés de l'enrobage sont de nature à aider l'homme du métier dans la sélection de ce dernier. En particulier, on peut citer l'homogénéité de l'enrobage, c'est-à-dire son épaisseur constante sur toute la surface du substrat, la facilité d'application, l'aptitude à pouvoir être lui-même recouvert d'un autre enrobage de nature identique ou différente et son caractère hydrophobe.

Selon la nature du substrat et selon l'application envisagée, l'enrobage pourra être de nature très diverse.

On pourra choisir le polymère insoluble dans l'eau à pH inférieur à 5 parmi les polymères filmogènes habituellement utilisés pour contrôler la libération, et connus de l'homme du métier, tels que les alkyl celluloses, plus particulièrement l'éthylcellulose, la carboxyméthyléthyl cellulose (CMEC), la carboxyméthyl cellulose, les esters de cellulose tels que l'acétate de cellulose, l'acétate phtalate de cellulose, l'acétate butyrate de cellulose, l'acétate succinate de cellulose, l'acétate trimellitate de cellulose, l'acétate succinate d'hydroxypropylméthyl cellulose, le phtalate d'hydroxypropylméthyl cellulose, les copolymères d'acide (méth)acrylique tels que la famille des Eudragit L, FS, S, RL, RS, RD, NE de la Société Evonik, (Darmstadt) ou les produits équivalents d'autres fournisseurs, l'acétate phtalate de polyvinyle, la gomme shellac. On peut mettre en oeuvre un seul de ces polymères ou bien plusieurs.

Selon un mode de réalisation avantageux, le polymère insoluble dans l'eau à pH inférieur à 5 représente 30 à 100% des composés insolubles dans l'eau à pH inférieur à 5 de l'enrobage.

On pourra choisir les composés insolubles dans l'eau à pH inférieur à 5 parmi les cires et corps gras solides au moins jusqu'à 37°C, tels que les huiles végétales hydrogénées, le palmitate de glycéryle, le béhénate de glycéryle, ou parmi les plastifiants hydrophobes tels que le sebacate de dibutyle, le phtalate de dibutyle, le phtalate de diméthyle, le citrate de tributyle.

On pourra choisir les composés solubles dans l'eau parmi les excipients connus pour cette qualité, comme le lactose, le mannitol, le maltitol, les sucres, mais aussi des polymères tels que polyvinylpyrrolidone (PVP); les dérivés solubles de la cellulose tels que l'hydroxypropylméthyl cellulose (HPMC), la méthyl cellulose, l'hydroxyéthyl cellulose, l'hydroxyéthylméthyl cellulose, l'hydroxypropyl cellulose, la carboxyméthyl cellulose sodique; l'isomalt; la maltodextrine; les poloxamers; le polyéthylèneglycol; le polyvinylalcool; le copolymère vinylpyrrolidone-acétate de vinyle; la gomme xanthane; la gomme d'acacia; la gomme carraghenane; la gomme guar; la gomme de caroube; l'agar-agar ; le polydextrose ; les copolymères de méthylvinyl éther et d'anhydride maléique ou d'acide maléique, et leurs mélanges, ou parmi les plastifiants hydrophiles tels que le triacétate de glycéryle, le triéthylcitrate, les tensioactifs. Ces composés pourront bien entendu être utilisés en tant que composé soluble unique, ou plus préférentiellement en mélange.

L'enrobage peut en outre contenir des charges, tensio-actifs, colorants, etc. et tout autre excipient connu de l'homme de l'art.

Selon un mode de réalisation avantageux, l'enrobage appliqué sur le substrat peut être un enrobage habituellement utilisé comme enrobage à libération contrôlée. En particulier, ceux décrits dans les brevets EP 0 709 087, WO 03/030878, EP 1 524 968, EP 1 524 969 et PCT/FR2009/050719.

Ainsi, selon une variante de l'invention, l'enrobage comprend au moins un polymère P1 filmogène insoluble dans l'eau à pH inférieur à 5, au moins un polymère P2 soluble dans l'eau, au moins un plastifiant PL, et éventuellement au moins un agent tensioactif et/ou un lubrifiant.

Plus spécifiquement, cet enrobage comprend :

- 50 à 90 % d'un polymère P1 filmogène insoluble dans l'eau à pH inférieur à 5, choisi dans le groupe comprenant les dérivés non hydrosolubles de la cellulose, notamment l'éthylcellulose, l'acétate de cellulose, l'acétate butyrate de cellulose, les copolymères d'ammonio(méth)acrylate de type A ou B, les copolymères d'éthylène et d'acétate de vinyle, et leurs mélanges,
- 2 à 25 %, de préférence 5 à 15 %, d'un polymère P2 soluble dans l'eau choisi dans le groupe comprenant la polyvinylpyrrolidone (PVP); les dérivés solubles de la cellulose tels que l'hydroxypropylméthyl cellulose (HPMC), la méthyl cellulose, l'hydroxyéthyl cellulose, l'hydroxyéthylméthyl cellulose, l'hydroxypropyl cellulose, la carboxyméthyl cellulose sodique ; l'isomalt ; la maltodextrine ; les poloxamers ; le polyéthylèneglycol ; le polyvinylalcool ; le copolymère vinylpyrrolidone-acétate de vinyle ; la gomme xanthane ; la gomme d'acacia ; la gomme carraghenane ; la gomme guar ; la gomme de caroube ; l'agar-agar, le polydextrose, les copolymères de méthylvinyl éther et d'anhydride maléique ou d'acide maléique et leurs mélanges, de préférence ce polymère étant la polyvinylpyrrolidone ;

- 2 à 20 %, de préférence 4 à 15 % de plastifiant PL choisi dans le groupe comprenant les esters de glycérol, les phtalates, les citrates, les sébacates, notamment le sébacate de dibutyle, les esters d'alcool cétylique, l'huile de ricin, le poly éthylène glycol et leurs mélanges ;
- 0 à 20 %, de préférence 2 à 15 %, de lubrifiant et/ou agent tensioactif choisi dans le groupe comprenant le stéarate de magnésium ou l'huile de ricin hydrogénée polyoxyéthylénée, et leurs mélanges.

Plus particulièrement, dans cet enrobage, P1 est choisi dans le groupe comprenant l'éthylcellulose, l'acétate de cellulose, l'acétate butyrate de cellulose, les copolymères d'ammonio(méth)acrylate de type A ou B, les copolymères d'éthylène et d'acétate de vinyle, P2 est de préférence une polyvinylpyrrolidone et le plastifiant PL est de préférence l'huile de ricin ou le sébacate de dibutyle.

A titre d'exemple, un tel enrobage peut comprendre 50 à 90 % d'éthylcellulose, 2 à 25 % de polyvinylpyrrolidone et 2 à 20 % d'huile de ricin.

Selon une autre variante de l'invention, l'enrobage comprend un polymère hydrophile A porteur de groupements ionisés à pH neutre et un composé B hydrophobe, cristallin à l'état solide et ayant une température de fusion $T_{fB} \geq 40°C$, de préférence $T_{fB} \geq 50°C$ et plus préférentiellement encore telle que $40°C \leq T_{fB} \leq 90°C$, le rapport pondéral B/A étant compris entre 0,25 et 1,5, de préférence entre 0,5 et 1.

Plus spécifiquement, dans cet enrobage, A est choisi dans le groupe comprenant les dérivés de la cellulose : l'acétate phtalate de cellulose, le phtalate d'hydroxypropylméthyl cellulose, l'acétate succinate d'hydroxypropylméthyl cellulose ; les copolymères d'acide (méth)acrylique et d'ester alkylique (méthylique) d'acide (méth)acrylique (EUDRAGIT ® S ou L) et leurs mélanges, et B est choisi dans le groupe comprenant les huiles végétales hydrogénées, les triglycérides, et leurs mélanges. Les triglycérides étant des produits naturels modifiés, il est entendu que les triglycérides peuvent contenir minoritairement notamment des mono- et/ou diglycérides.

Selon une autre variante, les quantités de P1, P2 et PL satisfont aux caractéristiques suivantes : la fraction massique en poids sec de P1 par rapport à la masse totale de l'enrobage est comprise entre 40 et 90%, la fraction massique en poids sec P2/P1+P2 est comprise entre 15 et 60% et la fraction massique en poids sec PL/P1+PL est comprise entre 1 et 30%.

Selon encore une autre variante, l'enrobage comprend un matériau composé d'au moins :

- 10 à 75 % en poids par rapport au poids total dudit enrobage d'au moins un polymère A insoluble dans l'eau à pH<5,
- 25 à 90 % en poids par rapport au poids total dudit enrobage d'au moins un polymère B possédant une valeur de pH de solubilisation variant dans la plage de pH de 5 à 7 et
- 0 à 25 % en poids par rapport au poids total dudit enrobage d'au moins un plastifiant,

lesdits polymères A et B étant présents dans un rapport pondéral polymère(s) B/polymère(s) A au moins égal à 0,25.

Le polymère A est choisi parmi l'éthylcellulose, l'acétate butyrate de cellulose, l'acétate de cellulose, les copolymères d'ammonio (méth)acrylate, de type A ou de type B, les esters d'acides poly(méth)acryliques, et leurs mélanges et le polymère B est choisi parmi le(s) copolymère(s) d'acide méthacrylique et de méthacrylate de méthyle, le(s) copolymère(s) d'acide méthacrylique et d'acrylate d'éthyle, les dérivés cellulosiques tels que l'acétate phtalate de cellulose, l'acétate succinate de cellulose, l'acétate trimellilate de cellulose, le phtalate d'hydroxypropylméthyl cellulose, l'acétate succinate d'hydroxypropylméthyl cellulose, la gomme shellac, l'acétate phtalate de polyvinyle, et leurs mélanges.

De façon préférée, cet enrobage est formé d'au moins un mélange d'éthylcellulose, d'acétate butyrate de cellulose ou de copolymère d'ammonio (méth)acrylate de type « A » ou « B » avec au moins un copolymère d'acide méthacrylique et d'acrylate d'éthyle ou d'un copolymère d'acide méthacrylique et de méthacrylate de méthyle ou un de leurs mélanges.

L'enrobage est appliqué par tout procédé classiquement utilisé pour l'enrobage de particules et notamment par pulvérisation (spray coating) en lit d'air fluidisé.

Le procédé conforme à l'invention comprend une étape 3/ de séchage des particules obtenues après l'extraction du substrat.

Selon un mode de réalisation particulier, l'étape 3/ de séchage est conduite en deux séquences, une première séquence de séchage à l'étuve ventilée puis une deuxième séquence de séchage en lit d'air fluidisé.

Le fait d'utiliser un tel séchage en deux séquences permet de commencer le séchage de façon lente et douce sans entraîner de chocs entre les particules creuses afin de limiter tout risque de déchirure de la coque formée par l'enrobage autour du substrat qui a été dissous.

Une fois que l'enrobage ou coque est solidifié, les particules creuses peuvent être soumises à des chocs sans risquer de déchirure. Le séchage peut donc être terminé dans un sécheur à lit d'air fluidisé.

L'invention porte également sur les particules creuses pouvant être obtenues selon le procédé décrit précédemment.

Les particules creuses selon l'invention présentent une résistance mécanique telle que déterminée par leur friabilité mesurée par un test de friabilité (donné ci-après dans le chapitre TESTS), inférieure à 30 %, de préférence inférieure à 20 %, et plus préférentiellement inférieure à 10 %.

Une telle résistance mécanique est nécessaire pour que les particules creuses résistent aux chocs et diverses contraintes qu'elles devront subir au cours de leurs utilisations.

Un autre objet de l'invention concerne l'utilisation desdites particules creuses comme supports d'une matière active ou principe actif alimentaire, cosmétique, phytopharmaceutique ou pharmaceutique, ladite matière active étant éventuellement protégée par un enrobage à libération contrôlée.

De telles particules sont représentées en D et F sur la Figure 1. En D, est représentée une particule creuse 8 sur laquelle est déposée une couche d'actif 10. En F, est représentée une particule creuse comprenant une couche d'actif 10 recouverte d'un enrobage à libération contrôlée 14. Sur ce schéma, est disposée une couche de protection 12 séparant la couche d'actif 10 de l'enrobage à libération contrôlée 14, cette couche de protection étant optionnelle.

Le principe actif est disposé sur la surface du coeur flottant, éventuellement à l'aide d'un liant, formant ainsi une couche de principe actif qui sera recouverte de l'enrobage à libération contrôlée. Ce liant permet une meilleure adhésion du principe actif sur le support neutre. Parmi les différents liants connus de l'homme de l'art, les polymères hydrosolubles sont utilisés selon un mode de réalisation avantageux de cette invention, tels que les polymères d'hydroxypropyl cellulose (Klucel de la société Aqualon), d'hydroxypropylméthyl cellulose (Methocel de BASF), de povidone (Plasdone d'ISP ou Kollidon de BASF), les polysaccharides, les gommes d'acacia, d'Agar, de Caroube et semblables. La nature du liant sélectionné pourra dépendre de sa compatibilité chimique avec le principe actif. D'un point de vue quantitatif ce liant est utilisé dans des proportions de 5 à 95%, de préférence de 5 à 50% et plus préférentiellement encore de 5 à 20% de la couche de principe actif. La couche de principe actif peut également contenir d'autres excipients classiquement utilisés par l'homme du métier. On peut citer notamment les agents dispersants, les tensio-actifs, les conservateurs, les tampons, les agents protecteurs, les charges, les colorants, et leurs mélanges.

Les particules creuses pourront être mises en oeuvre dans un milieu liquide où elles pourront flotter.

Les particules creuses selon l'invention présentent avantageusement une flottabilité F telle que mesurée selon le test de flottaison décrit ci-après dans le chapitre TESTS, supérieure ou égale à 50 % après au moins 1 h, de préférence supérieure ou égale à 50 % après au moins 4 h.

Leur masse volumique apparente est alors inférieure ou égale à 0,6 g/mL, de préférence inférieure ou égale à 0,5 g/mL, et plus préférentiellement encore inférieure ou égale à 0,4 g/mL.

De préférence, le diamètre moyen équivalent en volume mesuré à 0,1 bar du substrat utilisé dans le procédé selon l'invention est supérieur ou égal à 50 $\mu$m, de préférence compris entre 50 $\mu$m et 1000 $\mu$m, ou encore préférentiellement compris entre 50 $\mu$m et 500 $\mu$m. L'invention porte alors sur des particules enrobées comprenant les particules creuses décrites précédemment sur la surface desquelles est déposée au moins une substance active, éventuellement recouverte d'un enrobage à libération contrôlée. Selon un mode de réalisation particulier, ces particules enrobées sont conçues pour pouvoir flotter.

Afin de permettre cette flottaison, la quantité d'enrobage à libération contrôlée ne doit pas être trop importante. Cette quantité est déterminée par le taux de pelliculage TP$_2$ qui est calculé selon l'équation :

$$TP_2 = \frac{\text{poids de l'enrobage à libération contrôlée}}{\text{poids total des particules enrobées}} \times 100$$

Ainsi, si l'on se réfère à la figure 1, on voit que TP$_2$ correspond au poids de la couche 14 sur le poids total de la microparticule creuse 13.

TP$_2$ est compris entre 5 et 50 %, de préférence 10 et 40 % et plus préférentiellement entre 15 et 30 %.

La masse volumique apparente des particules enrobées est alors inférieure ou égale à 0,7 g/mL, de préférence inférieure ou égale à 0,6 g/mL, et plus préférentiellement encore inférieure ou égale à 0,5 g/mL.

L'invention concerne également les formulations comprenant des particules enrobées comportant au moins un principe actif et éventuellement un enrobage à libération contrôlée dudit principe actif disposés à la surface des particules creuses décrites ci-dessus.

Dans ces formulations, les particules enrobées ont un diamètre moyen équivalent en volume mesuré à 0,1 bar compris entre 50 et 4000 $\mu$m, notamment compris dans l'une des plages suivantes : 50 à 500 $\mu$m, 500 à 1000 $\mu$m, 1000 à 4000 $\mu$m.

Une application particulièrement intéressante des compositions actives de l'invention est celle du domaine pharmaceutique où il peut être utile pour certaines substances actives d'augmenter leur temps de séjour dans l'estomac afin d'optimiser leur biodisponibilité.

Une solution déjà connue pour augmenter ce temps de séjour dans l'estomac est de mettre en oeuvre des compositions pharmaceutiques aptes à flotter à la surface du liquide gastrique. Des compositions présentant cette propriété sont décrites notamment dans la demande de brevet déposée ce jour par la Demanderesse sous le n° 09 53601.

Ainsi, la présente invention concerne une composition multiparticulaire telle que décrite précédemment comprenant une pluralité de particules selon l'invention dans laquelle le principe actif est un principe actif pharmaceutique.

## Principe actif pharmaceutique

De telles compositions pharmaceutiques sont particulièrement avantageuses pour les principes actifs présentant une fenêtre d'absorption courte ou présentant une absorption préférentielle dans l'estomac. Ils peuvent également présenter un avantage pour les principes actifs ayant une activité locale dans l'estomac.

## Enrobage à libération contrôlée

L'enrobage à libération contrôlée est appliqué sur les particules par des techniques connues de l'homme du métier, par exemple pour la technique de l'enrobage par pulvérisation (spray coating) en lit d'air fluidisé.

Tout type d'enrobage conférant une libération contrôlée peut être mis en oeuvre dans les compositions pharmaceutiques de l'invention. En particulier, on peut citer les enrobages décrits notamment dans les demandes de brevet EP 0 709 087, WO 03/030878, EP 1 524 968, EP 1 524 969 et PCT/FR2009/050719. L'homme du métier est capable d'ajuster la nature de l'enrobage et le taux de pelliculage pour obtenir le profil de dissolution souhaité.

Ainsi, selon une variante de l'invention, l'enrobage à libération contrôlée comprend :

- au moins un polymère P1 filmogène insoluble dans l'eau choisi dans le groupe comprenant les dérivés non hydrosolubles de la cellulose, notamment l'éthylcellulose, l'acétate de cellulose, l'acétate butyrate de cellulose, les copolymères d'ammonio(méth)acrylate de type A ou B, les copolymères d'éthylène et d'acétate de vinyle et leurs mélanges,
- au moins un polymère P2 soluble dans l'eau choisi dans le groupe comprenant la polyvinylpyrrolidone (PVP) ; les dérivés solubles de la cellulose tels que l'hydroxypropylméthyl cellulose (HPMC), la méthyl cellulose, l'hydroxyéthyl cellulose, l'hydroxyéthylméthyl cellulose, l'hydroxypropyl cellulose, la carboxyméthyl cellulose sodique ; l'isomalt; la maltodextrine ; les poloxamers ; le polyéthylèneglycol ; le polyvinylalcool ; le copolymère vinylpyrrolidone-acétate de vinyle ; la gomme xanthane ; la gomme d'acacia ; la gomme carraghenane ; la gomme guar ; la gomme de caroube ; l'agar-agar, le polydextrose, les copolymères de méthylvinyl éther et d'anhydride maléique ou d'acide maléique et leurs mélanges, de préférence ce polymère étant la polyvinylpyrrolidone,

- au moins un plastifiant PL choisi dans le groupe comprenant les esters de glycérol, les phtalates, les citrates, les sébacates, notamment le dibutylsébacate, les esters d'alcool cétylique, l'huile de ricin, le polyéthylène glycol et leurs mélanges, et
- éventuellement au moins un agent tensioactif et/ou un lubrifiant choisi dans le groupe comprenant le stéarate de magnésium, l'huile de ricin hydrogénée polyoxyéthylénée, et leurs mélanges. Plus spécifiquement, cet enrobage peut comprendre :
- 50 à 90 % de polymère P1 filmogène insoluble dans l'eau,
- 2 à 25 %, de préférence 5 à 15 %, de polymère P2 soluble dans l'eau,
- 2 à 20 %, de préférence 4 à 15 % de plastifiant PL et
- 0 à 20 %, de préférence 2 à 15 % de lubrifiant et/ou agent tensioactif

Plus particulièrement, dans cet enrobage, P1 est choisi dans le groupe comprenant l'éthylcellulose, l'acétate de cellulose, l'acétate butyrate de cellulose, les copolymères d'ammonio(méth)acrylate de type A ou B, les copolymères d'éthylène et d'acétate de vinyle, P2 est de préférence une polyvinylpyrrolidone et le plastifiant PL est de préférence l'huile de ricin ou le sébacate de dibutyle.

A titre d'exemple, un tel enrobage peut comprendre 50 à 90 % d'éthylcellulose, 2 à 25 % de polyvinylpyrrolidone et 2 à 20 % d'huile de ricin.

Selon une autre variante de l'invention, l'enrobage à libération contrôlée comprend un polymère hydrophile A porteur de groupements ionisés à pH neutre et un composé B hydrophobe, cristallin à l'état solide et ayant une température de fusion $T_{fB} \geq 40°C$, de préférence $T_{fB} \geq 50°C$ et plus préférentiellement encore telle que $40°C \leq T_{fB} \leq 90°C$, le rapport pondéral B/A étant compris entre 0,25 et 1,5, de préférence entre 0,5 et 1.

Cet enrobage permet une libération du principe actif qui est à la fois retardée et contrôlée. Plus spécifiquement, dans cet enrobage, A est choisi dans le groupe comprenant les dérivés de la cellulose : acétate phtalate de cellulose, phtalate d'hydroxypropyl méthylcellulose, acétate succinate d'hydroxypropyl méthylcellulose; les copolymères d'acide (méth)acrylique et d'ester alkylique (méthylique) d'acide (méth)acrylique (EUDRAGIT ® S ou L) et leurs mélanges, et B est choisi dans le groupe comprenant les huiles végétales hydrogénées, les triglycérides, et leurs mélanges. Les triglycérides étant des produits naturels modifiés, il est entendu que les triglycérides peuvent contenir minoritairement notamment des mono- et/ou diglycérides.

Selon une autre variante, les quantités de P1, P2 et PL satisfont aux caractéristiques suivantes : la fraction massique en poids sec de P1 par rapport à la masse totale de l'enrobage est comprise entre 40 et 90 %, la fraction massique en poids sec P2/P1+P2 est comprise entre 15 et 60 % et la fraction massique en poids sec PL/P1+PL est comprise entre 1 et 30 %.

Selon encore une autre variante, l'enrobage à libération contrôlée comprend un matériau composé d'au moins :

- 10 à 75 % en poids par rapport au poids total dudit enrobage d'au moins un polymère A insoluble dans l'eau,
- 25 à 90 % en poids par rapport au poids total dudit enrobage d'au moins un polymère B possédant une valeur de pH de solubilisation variant dans la plage de pH de 5 à 7 et
- 0 à 25 % en poids par rapport au poids total dudit enrobage d'au moins un plastifiant,

lesdits polymères A et B étant présents dans un rapport pondéral polymère(s) B/polymère(s) A au moins égal à 0,25.

Le polymère A est choisi parmi l'éthylcellulose, l'acétate butyrate de cellulose, l'acétate de cellulose, les copolymères d'ammonio (méth)acrylate, de type A ou de type B, les esters d'acides poly(méth)acryliques, et leurs mélanges et le polymère B est choisi parmi le(s) copolymère(s) d'acide méthacrylique et de méthacrylate de méthyle, le(s) copolymère(s) d'acide méthacrylique et d'acrylate d'éthyle, les dérivés cellulosiques tels que l'acétate phtalate de cellulose, l'acétate succinate de cellulose, l'acétate trimellilate de cellulose, le phtalate d'hydroxypropylméthyl cellulose, l'acétate succinate d'hydroxypropylméthyl cellulose, la gomme shellac, l'acétate phtalate de polyvinyle, et leurs mélanges.

De façon préférée, cet enrobage est formé d'au moins un mélange d'éthylcellulose, d'acétate butyrate de cellulose ou de copolymère d'ammonio (méth)acrylate de type « A » ou « B » avec au moins un copolymère d'acide méthacrylique et d'acrylate d'éthyle ou d'un copolymère d'acide méthacrylique et de méthacrylate de méthyle ou un de leurs mélanges.

Selon un mode de réalisation particulier de l'invention, il peut être nécessaire d'appliquer entre la couche de principe actif et la couche d'enrobage à libération contrôlée, une couche supplémentaire ayant un rôle de protection du principe actif, notamment vis-à-vis de l'oxygène ou de l'humidité de l'air, et/ou d'un des composants de la couche d'enrobage. Cette couche supplémentaire pourra être appliquée à raison de quelques pourcents massiques par des techniques connues de l'homme du métier, par exemple par la technique de l'enrobage par pulvérisation (spray coating) en lit d'air fluidisé et consistera principalement en un polymère filmogène choisi parmi les polymères connus de l'homme du métier pour leur rôle de protection, tels que les polymères d'hydroxypropylméthyl cellulose ou de polyvinylalcool disponibles auprès de Colorcon Limited (Dartford UK).

Ainsi, selon le choix de l'enrobage et de sa composition, il sera possible d'ajuster la libération du principe actif indépendamment de sa capacité à flotter et donc de synchroniser au mieux cette libération avec le temps de séjour dans l'estomac et la cinétique d'absorption du principe actif. De plus, il est évident que ces systèmes possèdent des avantages par rapport à des systèmes basés sur des matrices gonflantes et gélifiantes, voire effervescentes : les dimensions des particules enrobées restent pratiquement constantes une fois immergées ce qui permet un contrôle et une reproductibilité de la libération, la flottaison est immédiate et ne nécessite pas un temps d'hydratation, de gonflement ou de réaction effervescente pendant lequel la forme ne flotte pas et risque d'être expulsée par le pylore.

La composition pharmaceutique selon la présente invention peut se présenter sous la forme de sachets, gélules, suspensions, comprimés ou comprimés orodispersibles pour administration orale. Outre les microparticules décrites précédemment, elle peut comprendre des excipients connus de l'homme du métier, tels que notamment des agents de compression, des liants, désintégrants, lubrifiants, viscosifiants, fluidifiants, colorants, édulcorants, etc.

La présente invention concerne également une méthode de traitement thérapeutique consistant essentiellement à administrer la composition de la présente invention par voie orale.

**Mode de préparation des compositions multiparticulaires**

Les compositions multiparticulaires selon l'invention peuvent être préparées selon un procédé comprenant les étapes successives suivantes :

a) sélection de particules creuses selon l'invention ;
b) application du principe actif, éventuellement en présence d'un liant, sur les particules creuses ;
c) éventuellement application d'un enrobage de protection sur les particules obtenues à l'étape b) ;
d) application de l'enrobage à libération contrôlée sur les particules obtenues à l'étape b) ou c).

Ce procédé de préparation est représenté de façon schématique sur la figure 1, chacun des produits obtenus à l'issue des étapes a) à c) étant représenté respectivement en C à F. Ainsi, les particules creuses 6 sont choisies. A la surface de celles-ci on dépose une couche 10 de principe actif et on obtient des particules creuses 9 comprenant un actif. A la surface de ces particules 9, on applique un enrobage de protection 12 pour obtenir les particules 11 représentées en E et finalement, on applique lors de l'étape d) un enrobage 14 à libération contrôlée et on obtient ainsi des particules creuses 13 comprenant un principe actif à libération contrôlée, représentées en F sur la figure 1.

Selon un mode de réalisation particulier de l'invention, il peut être nécessaire d'appliquer entre la couche de principe actif et la couche d'enrobage à libération contrôlée, une couche supplémentaire ayant un rôle de protection du principe actif, notamment vis-à-vis de l'oxygène ou de l'humidité de l'air, et/ou d'un des composants de la couche d'enrobage. Cette couche supplémentaire pourra être appliquée à raison de quelques pourcents massiques par des techniques connues de l'homme du métier, par exemple par la technique de l'enrobage par pulvérisation (spray coating) en lit d'air fluidisé et consistera principalement en un polymère filmogène choisi parmi les polymères connus de l'homme du métier pour leur rôle de protection, tels que les polymères d'hydroxypropylméthyl cellulose ou de polyvinylalcool disponibles auprès de Colorcon Limited (Dartford UK).

Le procédé peut éventuellement comprendre une étape supplémentaire de mélange avec des excipients, éventuellement suivie d'une étape de compression.

L'étape a) de sélection des particules creuses se fait selon les propriétés telles qu'elles sont mentionnées ci-dessus, à savoir leur masse volumique, leur taille, leur résistance mécanique.

Les étapes b), c) et d) sont réalisées à l'aide de techniques classiquement utilisées pour l'enrobage de particules, et notamment par enrobage par pulvérisation (spray coating) notamment en lit d'air fluidisé.

Ce procédé de préparation peut être mis en oeuvre facilement sur tout type d'installation d'enrobage de microparticules.

L'invention est décrite plus en détail encore à l'aide des exemples et figures ci-après qui sont non limitatifs et donnés uniquement à titre illustratif.

## TESTS

### *Mesure de la masse volumique apparente après tassement*

La méthode de mesure est basée sur l'essai du volume apparent décrit dans la pharmacopée européenne, édition 6.4, au chapitre 02.09.34. L'appareillage est constitué d'un appareil de tassement pouvant provoquer par minute 250 chutes d'une hauteur de 3 mm et d'une éprouvette de 250 mL graduée. 100,0 g de poudre sont introduits dans l'éprouvette sèche. Si le volume correspondant est supérieur à 250 $cm^3$ alors une masse de 50 g seulement est introduite. Une lecture du volume de la poudre est effectuée après que 1250 chutes ont été subies. Le rapport entre la masse introduite et le volume lu correspond à la masse volumique apparente tassée, exprimée en g/mL.

### *Mesure du diamètre moyen équivalent en volume D(4;3)*

Le diamètre moyen équivalent en volume est déterminé par diffraction laser ou analyse tamis selon l'échelle de taille à caractériser.

Jusqu'à une taille de 1000 $\mu m$, la distribution de tailles des particules est mesurée par diffraction laser à l'aide d'un appareil Mastersizer 2000 équipé d'un échantillonneur de poudre sèche (Malvern voie sèche module Scirocco 2000). A partir de la répartition granulométrique mesurée sur une large gamme, le diamètre moyen équivalent en volume ou D(4;3) est calculé selon la formule suivante :

$$D(4;3) = \Sigma(d^4) / \Sigma(d^3)$$

Pour une taille supérieure à 1000 $\mu m$, la méthode par tamisage analytique est utilisée. Le choix de tamis sera aisément fait par l'homme du métier par référence à la Pharmacopée Européenne, édition 6.4, chapitre 02.09.38.

### *Mesure de la friabilité*

Le test de friabilité consiste à mesurer le diamètre D(4;3) à l'aide d'un granulomètre laser par voie sèche à une pression de 0,1 bar (soit $D_{0,1}$), puis à une pression de 2 bars (soit $D_2$), la friabilité étant égale à :

$$\frac{D_{0,1} - D_2}{D_{0,1}} \times 100$$

### Mesure de la flottaison

Les performances des compositions pharmaceutiques ou de leurs constituants (microparticules enrobées, coeurs flottants) sont mesurées à l'aide d'un test de flottaison qui permet d'évaluer non seulement leur aptitude à flotter à la surface du milieu aqueux dans lesquelles elles sont plongées, mais également leur aptitude à remonter à la surface après avoir été plongées au fond du récipient par agitation du milieu. Un tel test consiste à déverser 300 à 500 mg de produit à tester dans 500 mL d'une solution d'acide chlorhydrique de normalité 0,1 N contenant 0,5 % en poids de tensioactif Tween 80, maintenus à 37°C pendant la durée du test. Un dispositif de dissolution tel qu'un dissolutest à palette décrit dans la pharmacopée européenne est utilisé. Le milieu est fortement agité à l'aide d'une spatule afin de permettre l'immersion totale du produit. Puis l'agitation est réglée à 50 tours/min pendant l'observation. Après 1 h, 4 h, 6 h et/ou 8 h, on retire les particules flottantes et on les pèse (on obtient une masse Mf) et on récupère les particules ayant coulé et on les pèse (on obtient une masse Mc), on calcule la valeur

$$F = (Mf / Mc+Mf) \times 100.$$

Les compositions pharmaceutiques de l'invention sont déclarées « systèmes flottants », si F est supérieure ou égale à 50 % après au moins 1 heure, de préférence après au moins 4 h, plus préférentiellement encore après au moins 6 h et encore plus préférentiellement après au moins 8 h.

### Mesure de la dissolution du carvédilol phosphate

Le suivi de la dissolution du carvédilol phosphate dans le milieu HCl 0,1 N est effectué par mesure de l'absorbance UV à la longueur d'onde de 285 nm dans des cellules de 0,5 cm en comparaison avec une courbe d'étalonnage préalablement établie. Une quantité de particules correspondant à 80 mg environ de carvedilol phosphate est introduite dans 900 mL de milieu, par bol d'appareil de dissolution, équipé de palettes d'agitation tournant à 100 rpm (appareil II de dissolution de la pharmacopée européenne) et maintenu à 37°C. Un prélèvement automatique est effectué à intervalles prédéterminés, et recyclé après lecture de l'absorbance.

### Mesure de la dissolution de la metformine HCl

Le suivi de la dissolution de la metformine HCl dans le milieu HCl 0,1 N + 0.5 % Tween 20 est effectué par mesure de l'absorbance UV à la longueur d'onde de 232 nm dans des cellules de 0,1 cm en comparaison avec une courbe d'étalonnage préalablement établie. Une quantité de particules correspondant à 500 mg environ de la metformine HCl est introduite dans 900 mL de milieu, par bol d'appareil de dissolution, équipé de palettes d'agitation tournant à 100 rpm (appareil II de dissolution de la pharmacopée européenne) et maintenu à 37°C. Un prélèvement automatique est effectué à intervalles prédéterminés, et recyclé après lecture de l'absorbance.

### EXEMPLES

Dans les exemples, les abréviations suivantes sont utilisées :

- Plasdone K29/32 : polyvinylpyrrolidone commercialisée par ISP
- Ethocel 7 premium : éthylcellulose commercialisée par Dow Chemicals
- TEC : citrate de triéthyle commercialisé par Morflex
- Eudragit L100-55 : copolymère d'acide méthacrylique/ méthacrylate de méthyle commercialisé par la société Evonik
- Lubritab : huile végétale hydrogénée/huile hydrogénée, commercialisé par la Société JRS Pharma
- Oil Blue N : anthraquinone commercialisée par la Société Sigma Aldrich

### Exemple 1 : Fabrication de supports sphériques creux

Dans un réacteur contenant 2250 g d'acétone et 1500 g d'isopropanol, on introduit sous agitation 222 g d'Ethocel 7 premium, 24 g d'huile de ricin, 24 g de Plasdone K29/32 et 30 g de stéarate de magnésium. La totalité de la préparation est pulvérisée sur 300 g de cristaux de sel (NaCl) à l'aide d'un appareil à lit d'air fluidisé Combi-Coata de GEA-Niro, soit un taux de pelliculage TP1 de 50 %. On obtient ainsi 582 g de microparticules. On observe les microparticules pleines obtenues au microscope électronique à balayage (grossissement x100) et on photographie l'une de ces microparticules entière (figure 2a) et en coupe (figure 2b).

Dans un bécher en inox de 1 L, on introduit 1 L de tampon $KH_2PO_4$ 0,05 M pH 7,4 maintenu à 37°C sous agitation d'une palette à 100 rpm. On verse dans ce milieu de dissolution 500 mg des microparticules obtenues précédemment.

Après 22 h, les microparticules sont tamisées sur tamis de 100 $\mu$m puis séchées à 40°C pendant 15 h dans une étuve ventilée. On observe les supports creux obtenus au microscope électronique à balayage (grossissement x100) et on photographie l'un de ces supports creux en entier (figure 3a) et en coupe (figure 3b). La totalité du sel contenu au départ s'est solubilisée, tout en maintenant l'intégrité de l'enrobage. Le diamètre est d'environ 1000 $\mu$m.

### Exemple 2 : Fabrication de supports sphériques creux

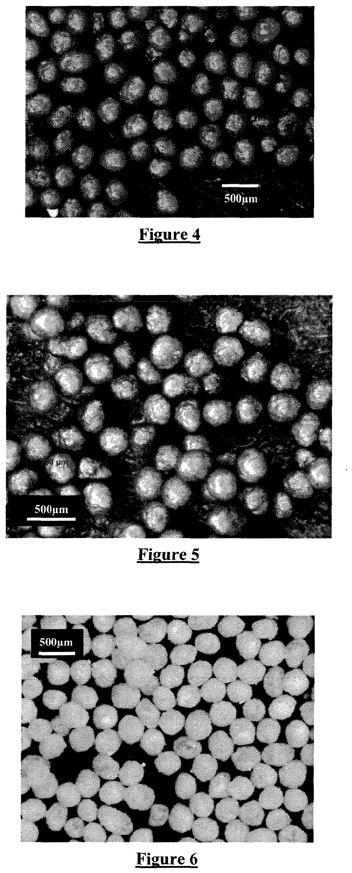

Dans un réacteur on introduit sous agitation 142,80 g d'Eudragit L100-55, 94,80 g de Lubritab, 2,40 g d'Oil Blue N dans 2160 g d'isopropanol chaud. Ce mélange est pulvérisé sur 560 g de Suglets 250-355 $\mu$m (sphères de sucre) à l'aide d'un appareil à lit d'air fluidisé GLATT GPCG1-1, jusqu'à un taux de pelliculage TP1 de 30 %. On obtient ainsi 795,5 g de microparticules pleines. On observe les microparticules pleines obtenues au microscope binoculaire (grossissement 1,6 x 1,6) et on les photographie (Figure 4).

La masse volumique apparente des microparticules pleines ainsi obtenues a été déterminée selon la méthode décrite ci-dessus et est égale à 0,814 g/cm³

Le diamètre moyen en volume à 0,1 bar et 2 bar et la friabilité des microparticules pleines sont obtenus par granulométrie laser au Malvern voie sèche module Scirocco 2000, conformément au test décrit plus haut.

| Pression (en bar) | Diamètre [4 ;3] (en $\mu$m) |
|---|---|
| 0,1 | 364 |
| 2 | 365 |
| Friabilité : | 0% |

Dans un bécher en inox de 10 L, on introduit 4 L de HCl 0,1 N et on ajoute 20 g de Lutrol F68. On verse dans ce milieu de dissolution 750 g des particules obtenues précédemment. Le milieu est maintenu à la température ambiante et agité à l'aide d'une hélice à 200 rpm pendant 24 h. On mesure l'indice de réfraction et la concentration en sucre dans la suspension au cours de la dissolution, au temps t = 0, t =18 h et t = 24 h.

Les résultats obtenus sont donnés dans le tableau ci-dessous :

| | Indice de réfraction | Concentration en sucre dans la suspension (en g/L) | % de sucre solubilisé |
|---|---|---|---|
| 0 | 1,3340 | 0 | 0,0 |
| 18h | 1,3350 | 7,58 | 5,8 |
| 24h | 1,3475 | 111,75 | 85,1 |

Après 24 h, les microparticules sont tamisées sur tamis de 100 $\mu$m puis séchées à 40°C pendant 2,5 h dans une étuve ventilée. En sortie d'étuve on récupère 598,9 g de particules que l'on sèche à nouveau en lit d'air fluidisé dans un appareil Glatt GPCG1.1 pendant 6 h 40 min. On obtient alors 303,9 g de supports creux que l'on tamise sur une toile de 630 $\mu$m. On obtient 285,4 g de supports creux de diamètre < 630 $\mu$m

On observe les supports creux obtenus au microscope binoculaire (grossissement 1,6 x 1,6) et on les photographie. La photographie obtenue est donnée sur la Figure 5. La grande majorité du sucre contenu au départ s'est solubilisé, tout en maintenant l'intégrité de la particule.

La masse volumique apparente des supports creux ainsi obtenus a été déterminée selon la méthode décrite ci-dessus et est égale à 0,361 g/cm³

Le diamètre moyen en volume à 0,1 bar et 2 bar et la friabilité des supports creux sont obtenus par granulométrie laser au Malvern voie sèche module Scirocco 2000, conformément au test décrit plus haut. La friabilité est faible et le diamètre moyen obtenu proche de celui des microparticules pleines.

| Pression (en bar) | Diamètre [4;3] (en μm) |
|---|---|
| 0,1 | 381 |
| 2 | 361 |
| Friabilité : | 5 % |

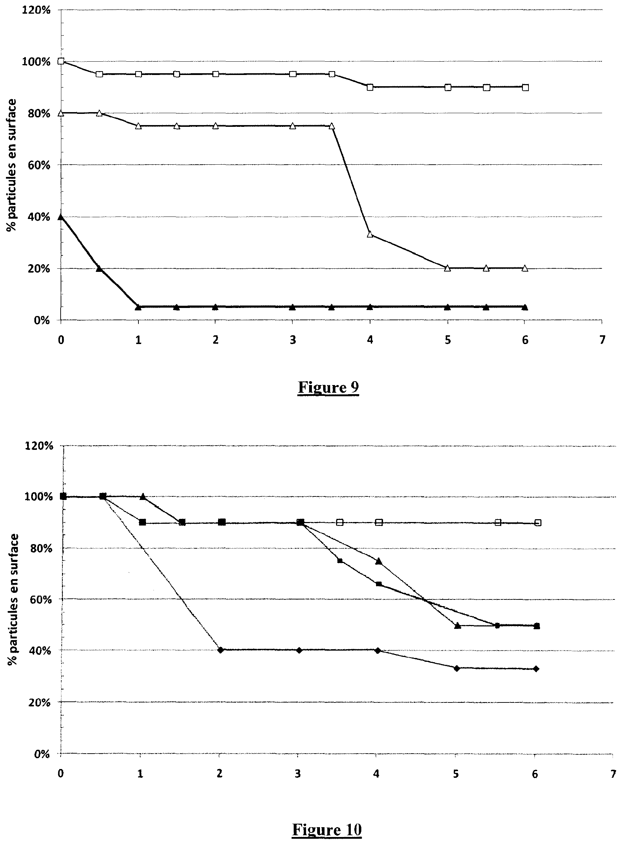

300 mg de supports creux obtenus ont été testés en flottaison dans HCl 0,1 N + 0,5 % en poids de Tween 80 selon le test décrit ci-dessus (tableau ci-dessous). Au bout de 6 h, 90 % des supports creux restent encore à la surface du milieu aqueux. La courbe en carrés blancs sur la Figure 9 représente la fraction de supports creux à la surface du milieu aqueux en fonction du temps.

| temps (h) | Fraction de supports creux à la surface du milieu aqueux |
|---|---|
| 0 | 100 % |
| 0,5 | 95 % |
| 1 | 95 % |
| 1,5 | 95 % |
| 2 | 95 % |
| 3 | 95 % |
| 3,5 | 95 % |
| 4 | 90 % |
| 5,5 | 90 % |
| 6 | 90 % |

### Exemple 3 : Fabrication de supports sphériques creux

Dans un bécher de 5 L on dissout 9,6 g de Crémophor RH40 et 24 g d'huile de ricin dans 1518 g d'acétone et 966 g d'isopropanol sous agitation. On ajoute 33,6 g de Plasdone K29-32, puis 172,8 g d'Ethocel 20. On ajoute ensuite 276 g d'eau. Ce mélange est pulvérisé sur 560 g de Suglets 250-355 μm (sphères de sucre) à l'aide d'un appareil à lit d'air fluidisé GLATT GPCG1-1, jusqu'à un taux de pelliculage de 30 %. On obtient ainsi 795,50 g de microparticules pleines.

On observe les microparticules pleines obtenues au microscope binoculaire (grossissement 1,6 x 1,6) et on les photographie. La photographie obtenue est donnée sur la figure 6.

Le diamètre moyen en volume à 0,1 bar et 2 bar et la friabilité des microparticules pleines sont obtenus par granulométrie laser au Malvern voie sèche module Sirocco 2000, conformément au test décrit plus haut.

| Pression (en bar) | Diamètre [4;3] (en μm) |
|---|---|
| 0,1 | 366 |
| 2 | 366 |
| Friabilité : | 0% |

La masse volumique apparente des microparticules ainsi obtenues est égale à 0,788 g/cm$^3$.

Le test de flottaison a été effectué dans un tampon pH 4,5 + 0,5 % Tween 80. La majorité des particules coule rapidement. La courbe en triangles noirs de la figure 9 représente la fraction de particules pleines à la surface du milieu aqueux en fonction du temps.

| temps (h) | Fraction de particules à la surface du milieu aqueux |
|---|---|
| 0 | 40 % |

(suite)

| temps (h) | Fraction de particules à la surface du milieu aqueux |
|-----------|------------------------------------------------------|
| 0,5 | 20 % |
| 1 | 5 % |
| 1,5 | 5 % |
| 2 | 5 % |
| 3 | 5 % |
| 3,5 | 5 % |
| 4 | 5 % |
| 5,5 | 5 % |
| 6 | 5 % |

On prépare 10 kg de tampon phosphate en mélangeant 68,05 g de $KH_2PO_4$ + 20 g Lutrol F68 + eau déminéralisée QSP 10 kg.

On verse dans 4 L de tampon phosphate, 700 g des particules obtenues précédemment. Le milieu est maintenu à la température ambiante et agité à l'aide d'une hélice à 250 rpm pendant 24 h. On mesure l'indice de réfraction et la concentration en sucre dans la suspension au cours de la dissolution, au temps t = 0, t = 18 h et t = 24 h.

Les résultats obtenus sont donnés dans le tableau ci-dessous :

| | Indice de réfraction | Concentration en sucre dans la suspension (en g/L) | % de sucre solubilisé |
|-----|----------------------|---------------------------------------------------|------------------------|
| 0 | 1,3340 | 0 | 0,0 |
| 5h | 1,3412 | 60,5 | 49,4 |
| 23h | 1,3470 | 108,8 | 88,8 |

Après 24 h, les microparticules sont tamisées sur tamis de 100 $\mu$m puis séchées à 50°C pendant 2,5 h dans une étuve ventilée. En sortie d'étuve on récupère 598,9 g de particules que l'on sèche à nouveau en lit d'air fluidisé dans un appareil Glatt GPCG1.1 pendant 1,5 h. On obtient alors 318,9 g de supports creux que l'on tamise sur tamis de 630 $\mu$m. On recueille 293,7 g de particules de diamètre < 630 $\mu$m.

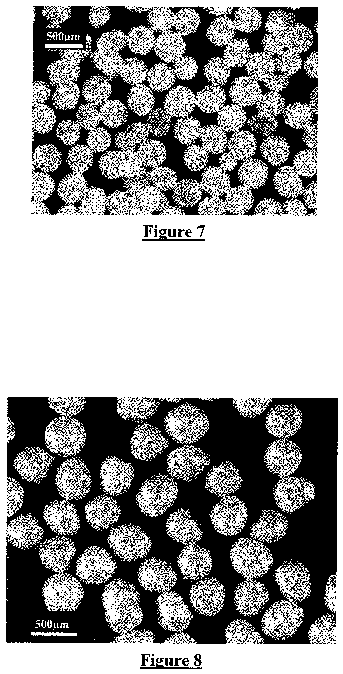

On observe les supports creux obtenus au microscope binoculaire et on les photographie. La photographie obtenue est donnée sur la figure 7. La grande majorité du sucre contenu au départ s'est solubilisé, tout en maintenant l'intégrité de la particule.

Les supports creux obtenus sont soumis à une granulométrie laser au Malvern voie sèche module Scirocco 2000, qui permet de déterminer leur diamètre moyen en volume à 0,1 bar et 2 bar, et leur friabilité. On obtient :

| Pression (en bar) | Diamètre (4;3) (en $\mu$m) |
|-------------------|----------------------------|
| 0,1 | 393 |
| 2 | 389 |
| Friabilité : | 1 % |

La masse volumique apparente des supports creux ainsi obtenus a été déterminée selon la méthode décrite ci-dessus et est égale à 0,322

Le test de flottaison a été effectué dans un tampon pH 4,5 + 0,5 % Tween 80. 75 % des particules restent à la surface du milieu aqueux pendant 3,5 h. La courbe en triangles blancs de la figure 9 représente la fraction de supports creux à la surface du milieu aqueux en fonction du temps.

| temps (h) | Fraction de particules à la surface du milieu aqueux |
|-----------|------------------------------------------------------|
| 0 | 80 % |

(suite)

| temps (h) | Fraction de particules à la surface du milieu aqueux |
|---|---|
| 0,5 | 80 % |
| 1 | 75 % |
| 1,5 | 75 % |
| 2 | 75 % |
| 3 | 75 % |
| 3,5 | 75 % |
| 4 | 33 % |
| 5,5 | 20 % |
| 6 | 20 % |

**Exemple 4 : Fabrication de particules flottantes à libération contrôlée de carvédilol phosphate à partir des supports creux de l'exemple 1**

Dans un bécher en inox de taille adaptée, on a introduit 480 g d'eau déminéralisée puis 720 g d'éthanol. Dans ce mélange solvant, on a introduit 80 g de Plasdone K29/32 et sous agitation, jusqu'à dissolution. Puis on a ajouté 320 g de carvédilol phosphate. Après homogénéisation, le mélange a été versé dans un bécher de 3 L.

Dans un réacteur à lit d'air fluidisé du type Glatt GPCG1-1, on a introduit 400 g de supports creux et on a pulvérisé 1600 g de la suspension de carvédilol phosphate précédemment obtenue. On a alors obtenu 712 g de granules creux comportant du carvédilol phosphate comme principe actif.

Sur ces derniers on a appliqué un enrobage à libération contrôlée de la façon suivante. Dans un bécher en inox on introduit 3 g d'huile de ricin et 8,25 g de TEC, puis 603,75 g d'éthanol sous agitation. On ajoute ensuite 7,5 g de Plasdone K29/32 puis 56,25 g d'Ethocel 7 premium. On ajoute ensuite 258,75 g d'eau déminéralisée.

Dans un réacteur à lit d'air fluidisé du type Glatt GPCG1-1, on introduit 300 g de granule de carvédilol phosphate et on pulvérise la solution précédemment obtenue de façon à obtenir un taux de pelliculage $TP_2$ de 20 %.

Les supports creux enrobés obtenus ont été photographiés sous microscope binoculaire (grossissement 1,6 x 1,6), la photographie résultante est donnée sur la figure 8.

La densité apparente tassée mesurée est de 0,472.

Le diamètre moyen en volume et la friabilité ont été mesurés. Les résultats sont donnés ci-après :

| Pression (en bar) | Diamètre (4 ;3) (en $\mu$m) |
|---|---|
| 0,1 | 417 |
| 2 | 399 |
| Friabilité | 4 % |

**Test de dissolution**

Le taux de dissolution de carvédilol phosphate en fonction du temps, mesuré selon le test décrit ci-dessus, a été mesuré pour les différentes particules à libération contrôlée de carvédilol phosphate préparées dans les exemples précédents.

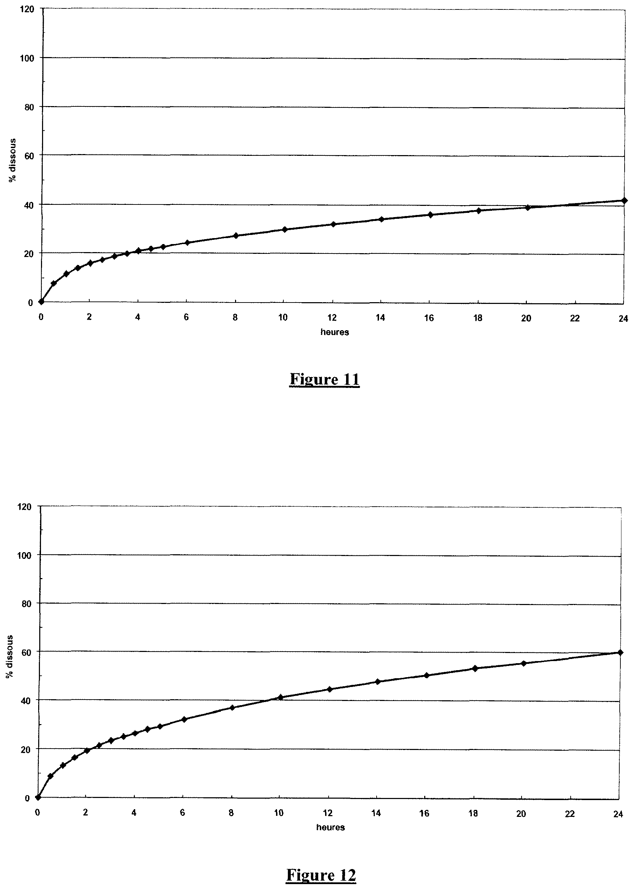

Les résultats sont présentés sur la figure 11 et montrent que la libération du carvédilol phosphate est prolongée.

**Test de flottaison**

On mesure la flottaison des particules conformément au test décrit ci-dessus. La fraction de particules à la surface du milieu aqueux en fonction du temps est présentée dans le tableau ci-dessous et illustrée sur la figure 10 (courbe avec carrés noirs).

| temps (h) | Fraction de particules à la surface du milieu aqueux |
|-----------|------------------------------------------------------|
| 0         | 100 %                                                |
| 0,5       | 100 %                                                |
| 1         | 90 %                                                 |
| 1,5       | 90 %                                                 |
| 2         | 90 %                                                 |
| 3         | 90 %                                                 |
| 3,5       | 75 %                                                 |
| 4         | 66 %                                                 |
| 5,5       | 50 %                                                 |
| 6         | 50 %                                                 |

**Exemple 5: Fabrication de particules flottantes à libération contrôlée de carvédilol phosphate à partir des supports creux de l'exemple 2**

On a procédé comme dans l'exemple 4 ci-dessus, en modifiant la valeur de $TP_2$, égal dans cet exemple à 15 %.

**Test de dissolution**

Le taux de dissolution de carvédilol phosphate en fonction du temps, mesuré selon le test décrit ci-dessus, a été mesuré pour les particules à libération contrôlée de carvédilol phosphate préparées dans l'exemple.
Les résultats sont présentés sur la figure 12 et montrent que la libération du carvédilol phosphate est prolongée.

**Test de flottaison**

On mesure la flottaison des particules conformément au test décrit ci-dessus. L'évolution de la fraction de particules à la surface du milieu aqueux en fonction du temps est présentée dans le tableau ci-dessous et illustrée dans la figure 10 (courbe avec carrés blancs).

| temps (h) | Fraction de particules à la surface du milieu aqueux |
|-----------|------------------------------------------------------|
| 0         | 100 %                                                |
| 0,5       | 100 %                                                |
| 1         | 90 %                                                 |
| 1,5       | 90 %                                                 |
| 2         | 90 %                                                 |
| 3         | 90 %                                                 |
| 3,5       | 90 %                                                 |
| 4         | 90 %                                                 |
| 5,5       | 90 %                                                 |
| 6         | 90 %                                                 |

**Exemple 6: Fabrication de particules flottantes à libération contrôlée de carvédilol phosphate à partir des supports creux de l'exemple 3**

Dans un bécher en inox de taille adaptée, on a introduit 480 g d'eau déminéralisée puis 720 g d'éthanol. Dans ce mélange solvant, on a introduit 80 g de Plasdone K29/32, et on a agité pendant 15 minutes à 600 rpm jusqu'à dissolution. Puis on a ajouté 320 g de carvédilol phosphate. Après homogénéisation, le mélange a été versé dans un bécher de 3 L.

Dans un réacteur à lit d'air fluidisé du type Glatt GPCG1-1, on a introduit 400 g de supports creux obtenus à l'exemple 3 et on a pulvérisé 1600 g de la suspension précédemment obtenue. On a alors obtenu des granules creux comportant du carvédilol phosphate comme principe actif.

Sur ces derniers on a appliqué un enrobage à libération contrôlée de la façon suivante. Dans un bécher en inox on introduit 3,00 g d'huile de ricin et 8,25 g de TEC, puis 603,75 g d'éthanol sous agitation. On ajoute ensuite 7,50 g de Plasdone K29/32 puis 56,25 g d'Ethocel 7 premium. On ajoute ensuite 258,75 g d'eau déminéralisée.

Dans un réacteur à lit d'air fluidisé du type Glatt GPCG1-1, on introduit 300 g de granules de carvédilol phosphate obtenus ci-dessus et on pulvérise la solution de façon à obtenir un taux de pelliculage $TP_2$ de 20 %.

La densité apparente tassée mesurée est de 0,515 g/cm$^3$.

Le diamètre moyen en volume et la friabilité ont été mesurés par granulométrie laser au Malvern voie sèche sur module Scirocco 2000. Les résultats sont donnés ci-après :

| Pression (en bar) | Diamètre (4;3) (en $\mu$m) |
|---|---|
| 0,1 | 467 |
| 2 | 464 |
| Friabilité | 1% |

### Test de dissolution

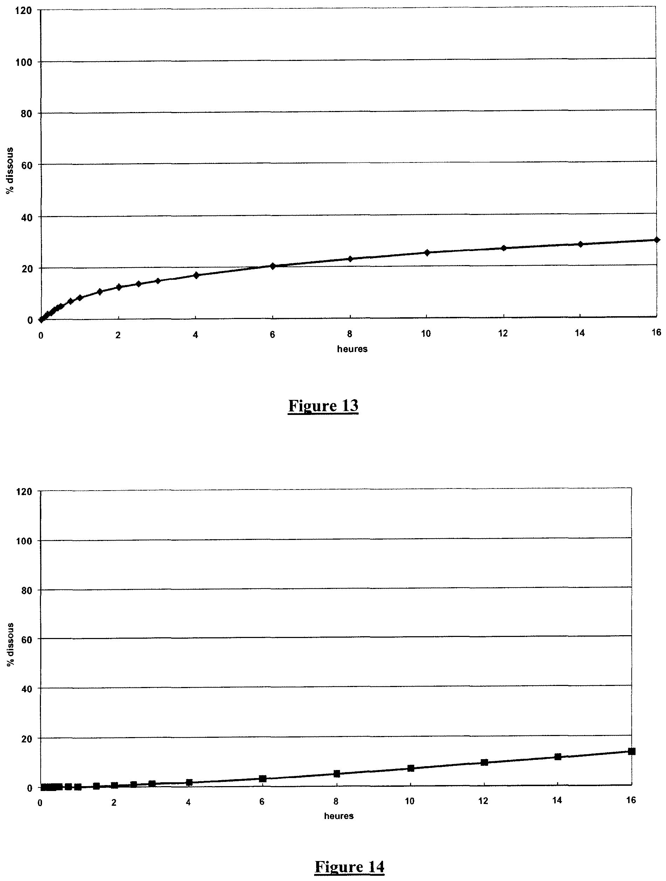

Le taux de dissolution de carvédilol phosphate en fonction du temps, mesuré selon le test décrit ci-dessus, a été mesuré pour les particules à libération contrôlée de carvédilol phosphate préparées dans cet exemple. Les résultats sont présentés sur la figure 13.

### Test de flottaison

On mesure la flottaison des particules conformément au test décrit ci-dessus. La fraction de particules à la surface du milieu aqueux en fonction du temps est présentée dans le tableau et illustré dans la figure 10 (courbe avec losanges noirs).

| temps (h) | Fraction de particules à la surface du milieu aqueux |
|---|---|
| 0 | 100 % |
| 0,5 | 100 % |
| 2 | 40 % |
| 3 | 40 % |
| 4 | 40 % |
| 5 | 33 % |
| 6 | 33% |

### Exemple 7: Fabrication de particules flottantes à libération contrôlée de carvédilol phosphate à partir des supports creux de l'exemple 3

Dans un bécher en inox de taille adaptée, on a introduit 480 g d'eau déminéralisée puis 720 g d'éthanol. Dans ce mélange solvant, on a introduit 80 g de Plasdone K29/32, et on a agité pendant 15 minutes à 600 rpm jusqu'à dissolution. Puis on a ajouté 320 g de carvédilol phosphate. Après homogénéisation, le mélange a été versé dans un bécher de 3 L.

Dans un réacteur à lit d'air fluidisé du type Glatt GPCG1-1, on a introduit 400 g de supports creux obtenus à l'exemple 4 et on a pulvérisé 1600 g de la suspension de précédemment obtenue. On a alors obtenu des granules creux comportant du carvédilol phosphate comme principe actif.

Sur 300 g de ces derniers on a appliqué un enrobage à libération contrôlée en procédant ainsi. Dans un réacteur on introduit 45 g d'Eudragit L100-55 et 30 g de Lubritab dans les solvants chauds sous agitation. Cette solution est pulvérisée à l'aide d'un appareil à lit d'air fluidisé GLATT GPCG1-1, jusqu'à un taux de pelliculage de 20 %. On obtient ainsi 268 g de particules de carvédilol phosphate à libération contrôlée.

*Le diamètre moyen en volume et la friabilité ont été mesurés par granulométrie laser au Malvern voie sèche sur module Scirocco 2000. Les résultats sont donnés ci-après :*

| Pression (en bar) | Diamètre (4;3) (en $\mu$m) |
|---|---|
| 0,1 | 464 |
| 2 | 456 |
| Friabilité | 8 % |

La densité apparente tassée mesurée est de 0,563 g/cm$^3$.

### Test de dissolution

Le taux de dissolution de carvédilol phosphate en fonction du temps, mesuré selon le test décrit ci-dessus, a été mesuré pour les particules à libération contrôlée de carvédilol phosphate préparées dans cet exemple. Les résultats sont présentés sur la figure 14.

### Test de flottaison

On mesure la flottaison des particules conformément au test décrit ci-dessus. La fraction de particules à la surface du milieu aqueux en fonction du temps est présentée dans le tableau et illustrée sur la figure 10 (courbe avec triangles noirs).

| temps (h) | Fraction de particules à la surface du milieu aqueux |
|---|---|
| 0 | 100 % |
| 0,5 | 100 % |
| 1 | 100 % |
| 2 | 90 % |
| 3 | 90 % |
| 4 | 75 % |
| 5 | 50 % |
| 6 | 50 % |

### Exemple 8 : Fabrication de particules flottantes à libération contrôlée de metformine HCl à partir des supports creux de l'exemple 2

Dans un bécher en inox de taille adaptée, on a laissé dissoudre sous agitation 400 g de metformine HCl dans 552,38 g d'eau déminéralisée.

Dans un réacteur à lit d'air fluidisé du type Glatt GPCG1-1, on a introduit 400 g de supports creux et on a pulvérisé la solution de metformine HCl précédemment obtenue. On a alors obtenu 732 g de granules creux comportant de la metformine HCl comme principe actif.

Sur ces derniers on a appliqué un enrobage apportant une libération contrôlée de la façon suivante. Dans un bécher en inox on a dissout 35,0 g d'Eudragit L100-55 et 52,5 g de Lubritab dans 787,5 g d'isopropanol sous agitation.

Dans un réacteur à lit d'air fluidisé du type Glatt GPCG1-1, on introduit 350 g de granules de metformine HCL obtenus à l'étape précédente et on pulvérise la solution préparée de façon à obtenir un taux de pelliculage TP$_2$ de 20 %.

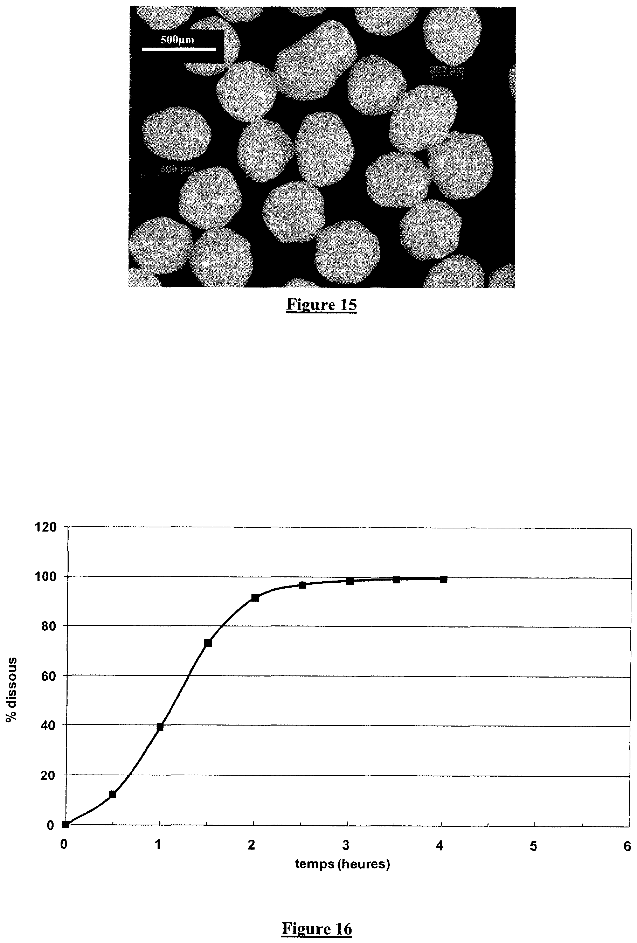

Les supports creux enrobés obtenus ont été photographiés sous microscope binoculaire (grossissement 3.2 x 1,6), la photographie résultante est donnée sur la figure 15.

La densité apparente tassée mesurée est de 0,52 g.cm$^{-3}$.

Le diamètre moyen en volume et la friabilité ont été mesurés. Les résultats sont donnés ci-après :

| Pression (en bar) | Diamètre (4 ;3) (en μm) |
|---|---|
| 0,1 | 429 |
| 2 | 413 |
| Friabilité | 4 % |

**Test de dissolution**

Le taux cumulé de dissolution de metformine HCL en fonction du temps a été mesuré selon le test décrit. Les résultats sont présentés sur la figure 16 et montrent que la libération du metformine HCL est prolongée.

**Test de flottaison**

On mesure la flottaison des particules conformément au test décrit ci-dessus. La fraction de particules à la surface du milieu aqueux en fonction du temps est présentée dans le tableau ci-dessous.

| temps (h) | Fraction de particules à la surface du milieu aqueux |
|---|---|
| 0 | 100 % |
| 1 | 100 % |
| 2 | 100 % |
| 4 | 97 % |
| 8 | 95 % |

**Exemple 9 : Fabrication de particules creuses**

Dans un réacteur on introduit sous agitation 124,67 g d'Ethocel 20 Premium, 23,23 g de Klucel EF, 11,71 g d'huile de ricin, 19,27 g d'acide tartrique, 10,01 g de Cremophor RH 40 dans un mélange de 1303,33 g d'acétone et 868,89 g d'isopropanol à température ambiante. Ce mélange est pulvérisé à l'aide d'un appareil à lit d'air fluidisé GLATT GPCG1-1 sur 1700 g de cristaux d'acide acétylsalicylique. On obtient ainsi 1784 g de microparticules pleines. Le diamètre moyen en volume à 0,1 bar des microparticules pleines obtenu par granulométrie laser est de 509 μm.

Dans un bécher en verre de 1 L, on introduit 1 L de HCl 0,1 N et on ajoute 5,0 g de Lutrol F68. On verse dans ce milieu de dissolution 5 g environ des particules obtenues précédemment. Le milieu est maintenu à 37°C et agité à l'aide d'une hélice à 100 tours/min pendant 48 h. Le milieu liquide est renouvelé entièrement après 24 h. Après 48 h, les microparticules sont tamisées sur tamis de 100 μm puis séchées à 40°C pendant 24 h dans une étuve ventilée. On obtient des microparticules creuses.

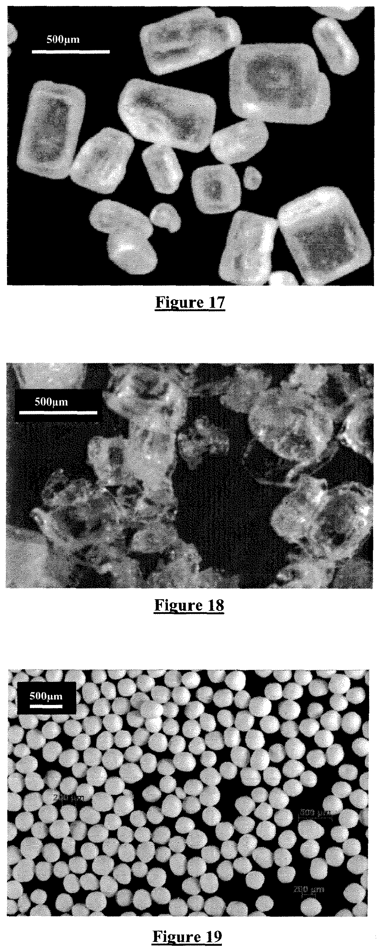

On observe les microparticules pleines et les particules creuses obtenues au microscope binoculaire (grossissement 3,2 x 1,6) et on les photographie. Les particules pleines sont représentées en Figure 17. Les particules creuses sont représentées en Figure 18.

**Exemple 10 : Fabrication de particules creuses**

Dans un réacteur on introduit sous agitation 156,92 g d'Ethocel 20 Premium, 8,89 g de Plasdone k29/32, 13,13 g d'huile de ricin, 21,60 g d'acide tartrique, 11,22 g de stéarate de magnésium dans un mélange de 1461,18 g d'acétone et 974,12 g d'isopropanol à température ambiante. Ce mélange est pulvérisé sur 1200,0 g de granulés de solubilité 3,5 g/l dans l'eau (particules de cellulose microcristalline et d'acide acétylsalicylique représentés en figure 19), à l'aide d'un appareil à lit d'air fluidisé GLATT GPCG1-1. On obtient ainsi 1319 g de microparticules pleines de diamètre moyen en volume à 0,1 bar égal à 322 μm (par granulométrie laser).

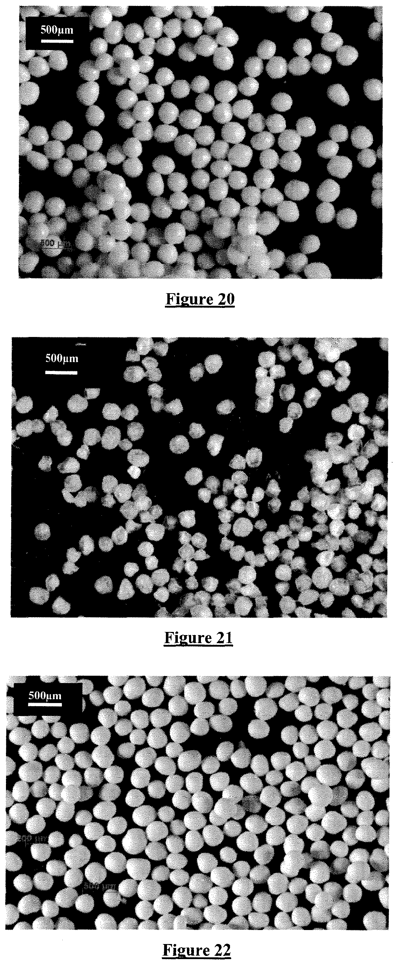

On observe les microparticules pleines obtenues au microscope binoculaire (grossissement 1,6 x 1,6) et on les photographie. Les particules pleines sont représentées en Figure 20.

Dans un bécher en verre de 1,0 L, on introduit 1 L de HCl 0,1 N et on ajoute 5,0 g de Lutrol F68. On verse dans ce milieu de dissolution environ 5 g des particules obtenues précédemment. Le milieu est maintenu à 37°C et agité à l'aide d'une hélice à 100 tours/min pendant 48 h. Le milieu liquide est renouvelé entièrement après 24 h. Les microparticules sont tamisées sur tamis de 100 μm puis séchées à 40°C.

On observe particules creuses obtenues au microscope binoculaire. Les particules creuses sont représentées en Figure 21. Les diamètres mesurés à l'aide du microscope binoculaire des particules creuses obtenues sont similaires à ceux des particules pleines.

## *Exemple 11 : Fabrication de particules creuses*

Dans un réacteur on introduit sous agitation 110,97 g d'Ethocel 20 Premium, 20,68 g de Klucel EF, 10,42 g d'huile de ricin, 17,15 g d'acide tartrique, 8,91 g de Cremophor RH 40 dans un mélange de 1160,11 g d'acétone et 773,41 g d'isopropanol à température ambiante. Ce mélange est pulvérisé à l'aide d'un appareil à lit d'air fluidisé GLATT GPCG1-1 sur 1700,0 g de granules de solubilité 3,5 g/l dans l'eau (particules de cellulose microcristalline et d'acide acétylsalicylique représentés en figure 22) et de 287 $\mu$m de diamètre. On obtient ainsi 1868 g de microparticules pleines de diamètre moyen en volume à 0,1 bar égal à 307 $\mu$m.

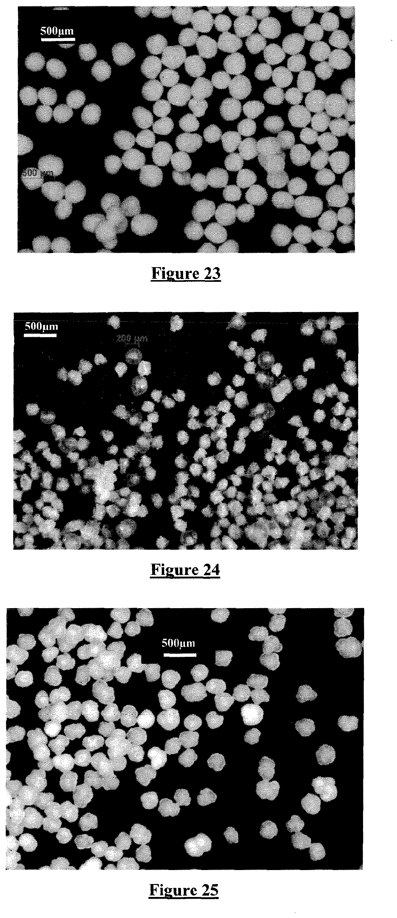

On observe les microparticules pleines obtenues au microscope binoculaire (grossissement 1,6 x 1,6) et on les photographie (Figure 23).

Dans un bécher en verre de 1,0 L, on introduit 1 L de HCl 0,1 N et on ajoute 5,0 g de Lutrol F68. On verse dans ce milieu de dissolution environ 5 g des particules obtenues précédemment. Le milieu est maintenu à 37°C et agité à l'aide d'une hélice à 100 tours/min pendant 48 h. Le milieu liquide est renouvelé entièrement après 24 h. Après 48 h, les microparticules sont tamisées sur tamis de 100 $\mu$m puis séchées à 40°C.

On observe les particules creuses obtenues au microscope binoculaire (grossissement 1,6 x 1,6) et on les photographie (Figure 24).

Les diamètres mesurés à l'aide du microscope binoculaire des particules creuses obtenues et des particules pleines sont similaires.

## *Exemple 12 : Fabrication de particules creuses*

Dans un réacteur on dissout sous agitation 57,12g d'Ethocel 20 Premium, 15,12 g de Plasdone k29/32, 8,40 g d'huile de ricin, 3,36 g de Cremophor RH 40 dans un mélange de 531,3 g d'acétone, 338,1 g d'isopropanol et 96,6 g d'eau. Ce mélange est pulvérisé à l'aide d'un appareil à lit d'air fluidisé GLATT GPCG1-1 sur 616,0 g de granules de 422 $\mu$m de diamètre, composés de mannitol, de Klucel EF et de paracétamol. On obtient ainsi 687 g de microparticules pleines de diamètre moyen en volume à 0,1 bar égal à 441 $\mu$m.

Dans un bécher en verre de 1,0 L, on introduit 1 L de HCl 0,1 N et on ajoute 5,0 g de Lutrol F68. On verse dans ce milieu de dissolution 5 g des particules obtenues précédemment. Le milieu est maintenu à 37°C et agité à l'aide d'une hélice à 100 tours/min pendant 48 h. Le milieu liquide est renouvelé entièrement après 24 h. Après 48 h, les microparticules sont tamisées sur tamis de 100 $\mu$m puis séchées à 40°C.

500 mg de particules creuses ont été testées en flottaison (tableau ci-dessous) dans HCl 0,1 N + 0,5 % en poids de Tween 80 selon le test décrit ci-dessus

| temps (h) | Fraction de particules à la surface du milieu aqueux |
|-----------|------------------------------------------------------|
| 0 | 100 % |
| 1 | 80 % |
| 2 | 60 % |
| 4 | 40 % |
| 6 | 40 % |

## *Exemple 13 : Fabrication de particules creuses*

Dans un réacteur on dissout sous agitation 97,92 g d'Ethocel 20 Premium, 25,92 g de Plasdone k29/32, 14,40 g d'huile de ricin, 5,76 g de Cremophor RH 40 dans un mélange de 910,8 g d'acétone, 579,6 g d'isopropanol et 165,6 g d'eau. Ce mélange est pulvérisé à l'aide d'un appareil à lit d'air fluidisé GLATT GPCG1-1 sur 656,0 g de granules de 119 $\mu$m de diamètre, composés de phosphate de calcium, hydroxypropyl cellulose et de paracétamol. On obtient ainsi 750 g de microparticules pleines.

Le diamètre moyen en volume à 0,1 bar des microparticules pleines obtenu par granulométrie laser est de 137 $\mu$m.

Dans un bécher en verre de 1,0 L, on introduit 1 L de HCl 0,1 N et on ajoute 5,0 g de Lutrol F68. On verse dans ce milieu de dissolution 5 g des particules obtenues précédemment. Le milieu est maintenu à 37°C et agité à l'aide d'une hélice à 100 tours/min pendant 48 h. Le milieu liquide est renouvelé entièrement après 24 h.

Après 48 h, les microparticules sont tamisées sur tamis de 100 $\mu$m puis séchées à 40°C dans une étuve ventilée. On obtient alors environ 1 g de particules creuses.

***Exemple 14 : Fabrication de particules creuses***

Dans un réacteur on dissout sous agitation 11,4 g de Cellulose Acetate Butyrate, 1,8 g de Plasdone k29/32, 1,5 g d'huile de ricin, 0,3 g de Cremophor RH 40 dans un mélange de 121,71 g d'acétone, 40,05 g d'éthanol et 12,94 g d'eau. Ce mélange est pulvérisé à l'aide d'un appareil à lit d'air fluidisé mini-GLATT sur 35,0 g de granules de 472 $\mu$m de diamètre (particules de cellulose microcristalline et de metformine). On obtient ainsi des microparticules pleines, de diamètre moyen en volume à 0,1 bar de 536 $\mu$m

Dans un bécher en verre de 1,0 L, on introduit 1 L de HCl 0,1 N et on ajoute 5,0 g de Lutrol F68. On verse dans ce milieu de dissolution 5 g des particules obtenues précédemment. Le milieu est maintenu à 37°C et agité à l'aide d'une hélice à 100 tours/min pendant 48 h. Le milieu liquide est renouvelé entièrement après 24 h. Après 48 h, les microparticules sont tamisées sur tamis de 100 $\mu$m puis séchées à 40°C.

On observe les particules creuses obtenues au microscope binoculaire. La grande majorité du substrat s'est solubilisée, tout en maintenant l'intégrité des particules. Les diamètres mesurés à l'aide du microscope binoculaire des particules creuses obtenues et des particules pleines sont similaires.

500 mg de particules creuses obtenues ont été testés en flottaison (tableau ci-dessous) dans HCl 0,1 N + 0,5 % en poids de Tween 80 selon le test décrit ci-dessus.

| temps (h) | Fraction de particules à la surface du milieu aqueux |
|-----------|------------------------------------------------------|
| 0 | 100 % |
| 1 | 90 % |
| 2 | 80 % |
| 4 | 60 % |
| 6 | 30 % |

***Exemple 15 : Fabrication de particules creuses***

Dans un réacteur on dissout sous agitation du Cellulose Acetate Butyrate, du Plasdone k29/32, du Cremophor RH 40 et de l'huile de ricin ou du triéthylcitrate ou du PEG, dans un mélange d'acétone, d'éthanol et d'eau, selon les quantités décrites dans le tableau ci dessous. La solution est pulvérisée à l'aide d'un appareil à lit d'air fluidisé de type mini-GLATT sur 30,0 g de substrat (particules de cellulose microcristalline et de metformine). On obtient ainsi des microparticules pleines de diamètre moyen en volume à 0,1 bar de 550-560 $\mu$m.

| Formulation | 15.a | 15.b | 15.c |
|-------------|------|------|------|
| Composants | Masse (g) | Masse (g) | Masse (g) |
| Cellulose Acétate Butyrate | 10,3 | 10,3 | 10,3 |
| Plasdone K29/32 | 1,0 | 1,0 | 1,0 |
| Cremophor RH40 | 0,3 | 0,3 | 0,3 |
| Huile de Ricin | 1,3 | - | - |
| Triéthylcitrate | - | 1,3 | - |
| PEG 400 | - | - | 1,3 |
| Acétone | 102,91 | 102,91 | 102,91 |
| Ethanol | 33,86 | 33,86 | 33,86 |
| Eau | 10,94 | 10,94 | 10,94 |

Dans un bécher en verre de 1,0 L, on introduit 1 L de HCl 0,1 N et on ajoute 5,0 g de Lutrol F68. On verse dans ce milieu de dissolution environ 5 g des particules obtenues précédemment. Le milieu est maintenu à 37°C et agité à l'aide d'une hélice à 100 tours/min pendant 48 h. Le milieu liquide est renouvelé entièrement après 24 h. Après 48 h, les microparticules sont tamisées sur tamis de 100 $\mu$m puis séchées à 40°C.

500 mg de supports creux obtenus ont été testés en flottaison (tableau ci-dessous) dans HCl 0,1 N + 0,5 % en poids de Tween 80 selon le test décrit ci-dessus. Les trois types de particules creuses obtenues flottent selon les critères définis.

| Formulation | 15.a | 15.b | 15.c |
|---|---|---|---|
| Temps (h) | % en surface | % en surface | % en surface |
| 0 | 100 | 100 | 100 |
| 1 | 50 | 75 | 75 |
| 2 | 25 | 40 | 60 |
| 4 | 10 | 30 | 50 |
| 6 | < 5 | 10 | 25 |

### Exemple 16 : Fabrication de particules creuses

Dans un réacteur on dissout sous agitation 117,0 g d'Eudragit L100-55 et 78,0 g de Cellulose Acétate Butyrate dans 2242,5 g d'acétone. Cette solution est pulvérisée dans un lit d'air fluidisé de type GLATT GPCG1-1 sur 455,0 g de substrat (particules de cellulose microcristalline et de metformine). On obtient ainsi 604 g de microparticules pleines. On observe les microparticules pleines obtenues au microscope binoculaire (grossissement 1,6 x 1,6) et on les photographie (Figure 25). Le diamètre moyen en volume à 0,1 bar des microparticules pleines obtenu par granulométrie laser est de 293 $\mu$m.

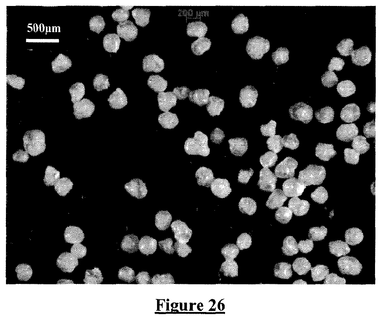

5 g de ces particules sont dispersés dans un bécher en verre contenant 1 L de HCl 0,1 N et 5,0 g de Lutrol F68. Le milieu est maintenu à 37°C et agité à l'aide d'une hélice à 100 tours/min pendant 48 h. Le milieu liquide est renouvelé entièrement après 24 h. Après 48 h, les microparticules sont tamisées sur tamis de 100 $\mu$m puis séchées à 40°C. On obtient alors quelques grammes de particules creuses. On observe les supports creux obtenus au microscope binoculaire (grossissement 1,6 x 1,6) et on les photographie (Figure 26). Les diamètres mesurés à l'aide du microscope binoculaire des particules creuses obtenues et des particules pleines sont similaires.

500 mg de supports creux obtenus ont été testés en flottaison (tableau ci-dessous) dans HCl 0,1 N + 0,5 % en poids de Tween 80 selon le test décrit ci-dessus. Au bout de 1 h, 90 % des supports creux restent encore à la surface du milieu aqueux, et 75 % environ après 6 h.

| temps (h) | Fraction de particules à la surface du milieu aqueux |
|---|---|
| 0 | 100 % |
| 1 | 90 % |
| 2 | 90 % |
| 4 | 90 % |
| 6 | 75 % |

### Exemple 17 : Fabrication de particules creuses

Dans un réacteur on dissout sous agitation 48,6 g d'Eudragit L100-55, 113,4 g de Cellulose Acétate Butyrate et 18,0 g de TEC dans 1863,0 g d'acétone et 207,0 g d'eau. On pulvérise ce mélange dans un lit d'air fluidisé de type GLATT GPCG1-1 sur 420,0 g de substrat (particules de cellulose microcristalline et de metformine). On obtient ainsi 503 g de microparticules pleines de diamètre moyen en volume à 0,1 bar de 288 $\mu$m.

Dans un bécher en verre de 1,0 L, on introduit 1 L de HCl 0,1 N et on ajoute 5,0 g de Lutrol F68. On verse dans ce milieu de dissolution 5 g des particules obtenues précédemment. Le milieu est maintenu à 37°C et agité à l'aide d'une hélice à 100 tours/min pendant 48 h. Le milieu liquide est renouvelé entièrement après 24 h.

Après 48 h, les microparticules sont tamisées sur tamis de 100 $\mu$m puis séchées à 40°C pendant 2,5 h. On obtient 1,5 g environ de particules creuses.

*Exemple 18 : Fabrication de particules creuses*

On procède de la même manière que dans l'exemple 19 mais on remplace les 18,0 g de TEC dans la formule par 18,0 g de dibutylphtalate. On obtient ainsi 518 g de microparticules pleines de diamètre moyen en volume à 0,1 bar de 289 $\mu$m.

Après l'étape de dissolution du substrat et de séchage des particules, conduites dans les mêmes conditions qu'à l'exemple précédent, on obtient environ 1,5 g de particules creuses.

**Revendications**

1. Procédé de préparation pour obtenir des particules creuses, lesdites particules creuses possédant une unique cavité vide entourée d'un enrobage, comprenant :

   1/ le dépôt par pulvérisation liquide, notamment en lit d'air fluidisé, sur un substrat partiellement soluble dans un milieu liquide M d'un enrobage composé de :

   - 40 à 100 % en poids d'au moins un composé insoluble dans l'eau à pH inférieur à 5, ledit composé insoluble dans l'eau à pH inférieur à 5 comprenant au moins un polymère, et
   - 60 à 0 % d'au moins un composé soluble dans l'eau, afin d'obtenir une particule comprenant ledit substrat enrobé d'une couche d'enrobage,

   2/ l'extraction dans le milieu M d'une partie du substrat, et
   3/ le séchage des particules obtenues à l'étape 2.

2. Procédé selon la revendication 1, **caractérisé par le fait que** le composé insoluble dans l'eau à pH inférieur à 5 comprend de 30 à 100 % de polymère.

3. Procédé selon les revendications 1 ou 2, **caractérisé par le fait que** le taux d'enrobage $TP_1$ est compris entre 5 et 90 %, de préférence entre 10 et 60 % et de préférence entre 15 et 40 %, le taux d'enrobage $TP_1$ étant calculé de la façon suivante :

$$TP_1 = \frac{\text{poids de l'enrobage}}{\text{poids de la particule enrobée}} \times 100,$$

les valeurs étant calculées par rapport à la matière sèche.

4. Procédé selon l'une quelconque des revendications 1 à 3, **caractérisé par le fait que** le substrat est en un matériau choisi dans le groupe comprenant le saccharose, le sucrose, les sphères de sucre, le mannitol, le maltitol, le lactose, le chlorure de sodium, le chlorure de potassium, et leurs mélanges.

5. Procédé selon l'une quelconque des revendications 1 à 4, **caractérisé en ce que** le diamètre moyen équivalent en volume mesuré à 0,1 bar du substrat est supérieur ou égal à 50 $\mu$m, de préférence compris entre 50 $\mu$m et 1000 $\mu$m, ou encore préférentiellement compris entre 50 $\mu$m et 500 $\mu$m.

6. Procédé selon l'une quelconque des revendications 1 à 5, **caractérisé par le fait que** le polymère insoluble dans l'eau à pH inférieur à 5 est choisi dans le groupe comprenant les alkyl celluloses, plus particulièrement l'éthylcellulose, la carboxyméthyléthyl cellulose (CMEC), la carboxyméthyl cellulose ; les esters de cellulose tels que l'acétate de cellulose, l'acétate phtalate de cellulose, l'acétate butyrate de cellulose, l'acétate succinate de cellulose, l'acétate trimellitate de cellulose, l'acétate succinate d'hydroxypropylméthyl cellulose, le phtalate d'hydroxypropylméthyl cellulose ; les copolymères d'acide (méth)acrylique ; l'acétate phtalate de polyvinyle ; la gomme shellac ; et leurs mélanges.

7. Procédé selon l'une quelconque des revendications 1 à 6, **caractérisé par le fait que** le composé insoluble dans l'eau à pH inférieur à 5 est choisi parmi les cires et corps gras solides au moins jusqu'à 37 °C, tels que les huiles végétales hydrogénées, le palmitate de glycéryle, le béhénate de glycéryle, ou parmi les plastifiants hydrophobes tels que le sébacate de dibutyle, le phtalate de dibutyle, le phtalate de diméthyle, le citrate de tributyle, et leurs mélanges.

8. Procédé selon l'une quelconque des revendications 1 à 7, **caractérisé par le fait que** le composé soluble dans l'eau est choisi dans le groupe comprenant le lactose, le mannitol, le maltitol, les sucres, mais aussi des polymères tels que polyvinylpyrrolidone (PVP); l'hydroxypropylméthyl cellulose (HPMC), la méthyl cellulose, l'hydroxyéthyl cellulose, l'hydroxyéthylméthyl cellulose, l'hydroxypropyl cellulose, la carboxyméthyl cellulose sodique; l'isomalt; la maltodextrine; les poloxamers; le polyéthylèneglycol; le polyvinylalcool; le copolymère vinylpyrrolidone-acétate de vinyle; la gomme xanthane; la gomme d'acacia; la gomme carraghenane; la gomme guar; la gomme de caroube; l'agar-agar ; le polydextrose ; les copolymères de méthylvinyl éther et d'anhydride maléique ou d'acide maléique, et leurs mélanges, ou parmi les plastifiants hydrophiles tels que le triacétate de glycéryle, le citrate de triéthyle, les tensioactifs et leurs mélanges.

9. Procédé selon l'une quelconque des revendications 1 à 8, **caractérisé en ce que** l'enrobage comprend:

   - au moins un polymère P1 filmogène insoluble dans l'eau à pH inférieur à 5,
   - au moins un polymère P2 soluble dans l'eau, et
   - au moins un plastifiant PL.

10. Procédé selon l'une quelconque des revendications 1 à 8, **caractérisé par le fait que** l'enrobage comprend :

   - un polymère hydrophile A porteur de groupements ionisés à pH neutre notamment choisi parmi l'acétate phtalate de cellulose, le phtalate d'hydroxypropylméthyl cellulose ; l'acétate succinate d'hydroxypropylméthyl cellulose, les copolymères d'acide (méth)acrylique et d'ester alkylique (méthylique) d'acide (méth)acrylique ou leurs mélanges et
   - un composé B hydrophobe, cristallin à l'état solide et ayant une température de fusion $T_{fB} \geq 40\ °C$, de préférence $T_{fB} \geq 50\ °C$ et plus préférentiellement encore telle que $40\ °C \leq T_{fB} \leq 90\ °C$, notamment choisi parmi les huiles végétales hydrogénées, les triglycérides ou leurs mélanges,
   - le rapport pondéral B/A étant compris entre 0,25 et 1,5, de préférence entre 0,5 et 1.

11. Particule creuse pouvant être obtenue par le procédé selon la revendication 9.

12. Particule creuse pouvant être obtenue par le procédé selon la revendication 10.

13. Utilisation de particules creuses selon les revendications 11 ou 12, comme supports d'au moins une matière active alimentaire, cosmétique, phytopharmaceutique ou pharmaceutique, ladite matière active étant éventuellement protégée par un enrobage à libération contrôlée.

14. Utilisation d'une particule creuse selon les revendications 11 ou 12, pour conférer à une microparticule comprenant ladite particule creuse une flottabilité F supérieure ou égale à 50 % après 1 heure selon le test de flottaison tel que défini ci-après:

   - déverser 300 à 500 mg de produit à tester dans 500 mL d'une solution d'acide chlorhydrique de normalité 0,1 N contenant 0,5 % en poids de tensioactif Tween® 80, maintenus à 37 °C pendant la durée du test, en utilisant un dispositif de dissolution tel qu'un dissolutest à palette décrit dans la pharmacopée européenne,
   - agiter fortement le milieu à l'aide d'une spatule afin de permettre l'immersion totale du produit,
   - puis régler l'agitation à 50 tours/min pendant l'observation,
   - après 1 h :

      ✔ retirer et peser les particules flottantes et
      ✔ récupérer et peser les particules ayant coulé,

   ladite valeur de flottabilité F étant calculée comme suit:

$$F = (Mf / Mc+Mf) \times 100$$

   où : Mf représente la masse des particules flottantes, et

Mc représente la masse des particules ayant coulé.

15. Particule enrobée comprenant une particule creuse selon les revendications 11 ou 12 sur la surface de laquelle est déposée au moins une substance active.

16. Particule enrobée selon la revendication 15, **caractérisée** en qu'elle est recouverte d'un enrobage à libération contrôlée.

17. Formulation comprenant les particules enrobées selon les revendications 15 ou 16.

**Patentansprüche**

1. Verfahren zur Herstellung von hohlen Partikel, wobei die hohlen Partikel einen einzigen leeren Hohlraum besitzen, der von einer Beschichtung umgeben ist, umfassend:

   1/ Abscheiden durch flüssige Zerstäubung, insbesondere im Wirbelschicht, auf einem Substrat, das in einem flüssigen Medium M löslich ist, einer Beschichtung, bestehend aus:

      - 40 bis 100 Gew.-% mindestens einer Komponente, die in Wasser bei einem pH von weniger als 5 unlöslich ist, wobei die Komponente, die in Wasser bei einem pH von weniger als 5 unlöslich ist, mindestens ein Polymer umfasst, und
      - 60 bis 0 % mindestens einer in Wasser löslichen Komponente, um ein Partikel zu erhalten, welches das Substrat, beschichtet mit einer Beschichtungsschicht, umfasst,

   2/ Extraktion eines Teils des Substrats in dem Medium M, und
   3/ Trocknen der in Schritt 2 erhaltenen Partikel.

2. Verfahren nach Anspruch 1, **dadurch gekennzeichnet, dass** die Komponente, die in Wasser bei einem pH von weniger als 5 unlöslich ist, 30 bis 100 % Polymer umfasst.

3. Verfahren nach den Ansprüchen 1 oder 2, **dadurch gekennzeichnet, dass** die Beschichtungsrate $TP_1$ zwischen 5 und 90 %, bevorzugt zwischen 10 und 60 % und bevorzugt zwischen 15 und 40 % beträgt, wobei die Beschichtungsrate $TP_1$ auf folgende Weise berechnet wird:

$$TP_1 = \frac{\text{Beschichtungsgewicht}}{\text{Gewicht des beschichteten Partikel}} \times 100 \text{ ,}$$

wobei die Werte in Bezug auf das Trockenmaterial berechnet werden.

4. Verfahren nach einem der Ansprüche 1 bis 3, **dadurch gekennzeichnet, dass** das Substrat aus einem Material ist, das ausgewählt wird aus der Gruppe umfassend Saccharose, Sucrose, Zuckersphären, Mannit, Maltit, Lactose, Natriumchlorid, Kaliumchlorid und ihren Mischungen.

5. Verfahren nach einem der Ansprüche 1 bis 4, **dadurch gekennzeichnet, dass** der volumenäquivalente mittlere Durchmesser, gemessen bei 0,1 bar, des Substrats größer oder gleich 50 $\mu$m ist, bevorzugt zwischen 50 $\mu$m und 1000 $\mu$m, oder noch bevorzugter zwischen 50 $\mu$m und 500 $\mu$m liegt.

6. Verfahren nach einem der Ansprüche 1 bis 5, **dadurch gekennzeichnet, dass** das Polymer, das in Wasser bei einem pH von weniger als 5 unlöslich ist, ausgewählt wird aus der Gruppe umfassend Alkylcellulosen, insbesondere Ethylcellulose, Carboxymethylethylcellulose (CMEC), Carboxymethylcellulose; Celluloseester, wie Celluloseacetat, Celluloseacetatphthalat, Celluloseacetatbutyrat, Celluloseacetatsuccinat, Celluloseacetattrimellitat, Hydroxypropylmethylcelluloseacetatsuccinat, Hydroxypropylmethylcellulosephthalat; (Meth)acrylsäure-Copolymere; Polyvinylacetatphthalat; Shellack-Gummi; und ihre Mischungen.

7. Verfahren nach einem der Ansprüche 1 bis 6, **dadurch gekennzeichnet, dass** die Komponente, die in Wasser bei einem pH von weniger als 5 unlöslich ist, ausgewählt wird aus Wachsen und Fettkörpern, die bis mindestens 37°C fest sind, wie hydrierten Pflanzenölen, Glycerylpalmitat, Glycerylbehenat, oder aus hydrophoben Weichmachern, wie Dibutylsebacat, Dibutylphthalat, Dimethylphthalat, Tributylcitrat, und ihren Mischungen.

8. Verfahren nach einem der Ansprüche 1 bis 7, **dadurch gekennzeichnet, dass** die in Wasser lösliche Komponente ausgewählt wird aus der Gruppe umfassend Lactose, Mannit, Maltit, Zucker, jedoch auch Polymere, wie Polyvinylpyrrolidon (PVP); Hydroxypropylmethylcellulose (HPMC), Methylcellulose, Hydroxyethylcellulose, Hydroxyethylmethylcellulose, Hydroxypropylcellulose, Natriumcarboxymethylcellulose; Isomalt; Maltodextrin; Poloxamere; Polyethylenglykol; Polyvinylalkool; Vinylpyrrolidon-Vinylacetat-Copolymer; Xanthan-Gummi; Akaziengummi; Carragheenangummi; Guar-Gummi, Johannisbrotgummi; Agar-Agar; Polydextrose; Copolymere von Methylvinylether und Maleinsäureanhydrid oder Maleinsäure, und ihre Mischungen, oder aus hydrophilen Weichmachern, wie Glyceryltriacetat, Triethylcitrat, Tensid und ihre Mischungen.

9. Verfahren nach einem der Ansprüche 1 bis 8, **dadurch gekennzeichnet, dass** die Beschichtung umfasst:

    - mindestens ein filmbildendes Polymer P1, das in Wasser bei einem pH von weniger als 5 unlöslich ist,
    - mindestens ein Polymer P2, das in Wasser löslich ist, und
    - mindestens einen Weichmacher PL.

10. Verfahren nach einem der Ansprüche 1 bis 8, **dadurch gekennzeichnet, dass** die Beschichtung umfasst:

    - ein hydrophiles Polymer A, das ionisierte Gruppen bei einem neutralen pH trägt, insbesondere ausgewählt aus Celluloseacetatphthalat, Hydroxypropylmethylcellulosephthalat; Hydroxypropylmethylcelluloseacetatsuccinat, Copolymeren von (Meth)acrylsäure und Alkylestern (Methylestern) von (Meth)acrylsäure oder ihren Mischungen, und
    - eine hydrophobe Komponente B, die im festen Zustand kristallin ist und eine Schmelztemperatur von $T_{fB} \geq$ 40°C, bevorzugt $T_{fB} \geq$ 50°C und noch bevorzugter wie $40°C \leq T_{fB} \leq 90°C$, aufweist, insbesondere ausgewählt aus hydrierten Pflanzenölen, Triglyceriden oder ihren Mischungen,
    - wobei das Gewichtsverhältnis B/A zwischen 0,25 und 1,5, bevorzugt zwischen 0,5 und 1, liegt.

11. Hohles Partikel, welches gemäß dem Verfahren nach Anspruch 9 erhalten werden kann.

12. Hohles Partikel, welches gemäß dem Verfahren nach Anspruch 10 erhalten werden kann.

13. Verwendung von hohlen Partikel nach den Ansprüchen 11 oder 12, als Träger mindestens eines aktiven alimentären, kosmetischen, phytopharmazeutischen oder pharmazeutischen Stoffs, wobei der aktive Stoff gegebenenfalls durch eine Beschichtung mit kontrollierter Freisetzung geschützt ist.

14. Verwendung eines hohlen Partikels nach den Ansprüchen 11 oder 12, um einem Mikropartikel, welches das hohles Partikel umfasst, einen Auftrieb F größer oder gleich 50 % nach 1 Stunde gemäß dem Flotationstest, wie nachstehend definiert, zu verleihen:

    - Füllen von 300 bis 500 mg des zu testenden Produkts in 500 ml einer Salzsäurelösung mit einer Normalität von 0,1 N, enthaltend 0,5 Gew.-% des Tensid Tween® 80, gehalten bei 37°C während der Dauer des Tests, unter Verwendung einer Lösungsvorrichtung, wie einer Platten-Dissolutest-Vorrichtung, die in dem europäischen Arzneibuch beschrieben ist,
    - heftiges Rühren des Mediums mit Hilfe eines Spachtels, um das gesamte Eintauchen des Produkts zu gestatten,
    - anschließend Regeln des Rührens bei 50 UpM während der Beobachtung,
    - nach 1 Stunde:

        ✔ Abziehen und Wiegen der schwimmenden Partikel, und
        ✔ Gewinnen und Wiegen der Partikel, die abgesunken sind,

wobei der Wert des Auftriebs F wie folgt berechnet wird:

$$F = (Mf/Mc+Mf) \times 100$$

wobei: Mf die Masse der schwimmenden Partikel repräsentiert, und
Mc die Masse der Partikel repräsentiert, die abgesunken sind.

15. Beschichtetes Partikel, umfassend ein hohles Partikel nach den Ansprüchen 11 oder 12, auf der Oberfläche von welchem mindestens eine aktive Substanz abgeschieden wird.

16. Beschichtetes Partikel nach Anspruch 15, **dadurch gekennzeichnet, dass** dieses mit einer Beschichtung mit gesteuerter Freisetzung bedeckt ist.

17. Formulierung, umfassend beschichtete Partikel nach den Ansprüchen 15 oder 16.

**Claims**

1. Process for the preparation of hollow particles comprising, said hollow particles having a single hollow cavity surrounded by a coating, comprising:

   1/ depositing by liquid spraying, in particular in a fluidized bed, onto a substrate partially soluble in a liquid medium M, a coating comprising:

   - 40 to 100 % by weight of at least one compound which is insoluble in water at a pH of less than 5, said compound which is insoluble in water at a pH of less than 5 comprising at least one polymer, and
   - 60 to 0 % of at least one water-soluble compound, thus obtaining a particle comprising said sbtrate surrounded by a coating layer,

   2/ extracting a part of the substrate into the medium M,
   3/ drying the particles obtained in stage 2.

2. Process according to claim 1, **characterized in that** the compound which is insoluble in water at a pH of less than 5 comprises from 30 to 100% of polymer.

3. Process according to claim 1 or 2, **characterized by** the fact that the coating ratio $TP_1$ is comprised between 5 and 90%, preferably between 10 and 60% and preferably between 15 and 40%, the coating ratio TP1 being calculated as follows:

$$TP1 = \frac{\text{weight of the coating}}{\text{weight of the coated particle}} \times 100 \ ,$$

the values being calculated relative to the dry matter.

4. Process according to any one of claims 1 to 3, **characterized in that** the substrate is of a material chosen from the group comprising saccharose, sucrose, sugar spheres, mannitol, maltitol, lactose, sodium chloride, potassium chloride, and mixtures thereof.

5. Process according to any one of claims 1 to 4, **characterized in that** the equivalent volume mean diameter of the substrate measured at 0.1 bar is greater than or equal to 50 $\mu$m, preferably comprised between 50 $\mu$m and 1000 $\mu$m, or even preferentially comprised between 50 $\mu$m and 500 $\mu$m.

6. Process according to any one of claims 1 to 5, **characterized by** the fact that the polymer which is insoluble in water at a pH of less than 5 is chosen from the group comprising the alkyl celluloses, more particularly ethylcellulose, carboxymethylethyl cellulose (CMEC), carboxymethyl cellulose ; the cellulose esters such as cellulose acetate, cellulose acetate phthalate, cellulose acetate butyrate, cellulose acetate succinate, cellulose acetate trimellitate, hydroxypropylmethyl cellulose acetate succinate, hydroxypropylmethyl cellulose phthalate ; the (meth)acrylic acid copolymers; polyvinyl acetate phthalate, shellac gum; and mixtures thereof.

7. Process according to any one of claims 1 to 6, **characterized in that** the compound which is insoluble in water at a pH of less than 5 is chosen from the waxes and fats which are solid at least up to 37°C, such as hydrogenated vegetable oils, glyceryl palmitate, glyceryl behenate, or from hydrophobic plasticizers such as dibutyl sebacate, dibutyl phthalate, dimethyl phthalate, tributyl citrate and mixtures thereof.

8. Process according to any one of claims 1 to 7, **characterized in that** the water-soluble compound is chosen from the group comprising lactose, mannitol, maltitol, sugars, but also polymers such as polyvinylpyrrolidone (PVP); hydroxypropylmethyl cellulose (HPMC), methyl cellulose, hydroxyethyl cellulose, hydroxyethylmethyl cellulose, hydroxypropyl cellulose, sodium carboxymethyl cellulose; isomalt; maltodextrin; poloxamers; polyethylene glycol; polyvinyl alcohol; vinyl pyrrolidone-vinyl acetate copolymer; xanthan gum; acacia gum; carragheenan gum; guar gum; carob gum; agar-agar; polydextrose; methylvinyl ether and maleic anhydride or maleic acid copolymers and mixtures thereof, or from hydrophilic plasticizers such as glyceryl triacetate, triethyl citrate, surfactants and mixtures thereof.

9. Process according to any one of claims 1 to 8, **characterized in that** the coating comprises:

   - - at least one film-forming polymer P1 insoluble in water at a pH of less than 5,
   - at least one water-soluble polymer P2, and
   - at least one plasticizer PL.

10. Process according to any one of claims 1 to 8, **characterized in that** the coating comprises :

   - a hydrophilic polymer A bearing ionized groups at neutral pH in particular chosen from cellulose acetate phthalate, hydroxypropylmethyl cellulose phthalate; hydroxypropylmethyl cellulose acetate succinate, copolymers of (meth)acrylic acid and (methyl) alkyl ester of (meth)acrylic acid or their mixtures and
   - a hydrophobic compound B which is crystalline in the solid state and has a melting point $T_{fB} \geq 40\ °C$, preferably $T_{fB} \geq 50\ °C$ and even more preferentially such that $40\ °C \leq T_{fB} \leq 90\ °C$, in particular chosen from hydrogenated vegetable oils, triglycerides or mixtures thereof,
   - the B/A weight ratio being comprised between 0.25 and 1.5, preferably between 0.5 and 1.

11. Hollow particle which can be obtained by the process according to claim 9.

12. Hollow particle which can be obtained by the process according to claim 10.

13. Use of hollow particles according to claim 11 or 12 as supports for at least one food, cosmetic, phytopharmaceutical or pharmaceutical active ingredient, said active ingredient being optionally protected by a controlled-release coating.

14. Use of a hollow particle according to claim 11 or 12, to confer upon a microparticle comprising said hollow particle a floatability F greater than or equal to 50 % after at least 1 hour according to the flotation test as defined below:

   - pouring 300 to 500 mg of product to be tested into 500 mL of a solution of hydrochloric acid of normality 0.1 N containing 0.5% by weight Tween® 80 surfactant, maintained at 37°C for the duration of the test, using a dissolution device such as a paddle dissolutest described in the European Pharmacopoeia,
   - vigorously stirring the medium using a spatula in order to allow the total immersion of the product,
   - then adjusting the stirring to 50 rpm during the observation,
   - after 1 hour:

      ✓ remove and weigh the floating particles and
      ✓ collect and weigh the particles which have sunk,

   said floatability F value is calculated as follows:

$$F= (Mf / Mc+Mf) \times 100$$

   wherein: Mf represents the mass of the floating particles, and
   Mc represents the mass of the particles which have sunk.

15. Coated particle comprising a hollow particle according to claim 11 or 12 on the surface of which at least one active ingredient is deposited.

**16.** Coated particle according to claim 15, **characterized in that** it is covered with a controlled-release coating.

**17.** Formulation comprising the coated particles according to claim 15 or 16.

**Figure 1**

**Fig 2a**

**Fig 2b**

**Fig 3a**

**Fig 3b**

**Figure 4**

**Figure 5**

**Figure 6**

**Figure 7**

**Figure 8**

**Figure 9**

**Figure 10**

**Figure 11**

**Figure 12**

**Figure 13**

**Figure 14**

**Figure 15**

**Figure 16**

**Figure 17**

**Figure 18**

**Figure 19**

**Figure 20**

**Figure 21**

**Figure 22**

**Figure 23**

**Figure 24**

**Figure 25**

**Figure 26**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 9118590 A
- WO 9722409 A
- WO 9920254 A
- EP 709087 A
- WO 03030878 A

- EP 1524968 A
- EP 1524969 A
- FR 2009050719 W
- EP 0709087 A

**Littérature non-brevet citée dans la description**

- **A. STREUBEL et al.** *International Journal of Pharmaceutics,* 2002, vol. 241, 279-292
- **N.J. JOSEPH et al.** *Journal of Controlled Release,* 2002, vol. 79, 71-79
- **Y. SENUMA.** *Biomaterials,* 2000, vol. 21, 1135-1144
- **JINGWEY XIE et al.** *Journal of Colloid and Interface Science,* vol. 302 (206), 103-112

- **R.TALUKDER ; R. FASSIHI.** *Drug Development and Industrial Pharmacy,* 2004, vol. 30 (4), 405-412
- **Y. MURATA et al.** *European Journal of Pharmaceutics and Biopharmaceutics,* 2000, vol. 50, 221-226